# EUROPÄISCHE PATENTSCHRIFT

(11) **EP 4 570 279 B1**
(45) Veröffentlichungstag und Bekanntmachung des Hinweises auf die Patenterteilung: **11.11.2026**
(21) Anmeldenummer: 24218619.5
(22) Anmeldetag: 10.12.2024
(51) Int. Cl.: A61M 1/16

(54) **VERFAHREN ZUM BESTIMMEN EINER NATRIUM- UND BICARBONATIONENKONZENTRATION EINER DIALYSIERFLÜSSIGKEIT, VERWENDUNG EINES VERFAHRENS ZUR PROPORTIONIERUNG DER DIALYSIERFLÜSSIGKEIT**
PROCESS FOR DETERMINING THE SODIUM AND BICARBONATE ION CONCENTRATION OF A DIALYSIS FLUID, USE OF A METHOD FOR PROPORTIONING DIALYSIS FLUID
PROCÉDÉ DE DÉTERMINATION D'UNE CONCENTRATION D'IONS SODIUM ET BICARBONATE D'UN LIQUIDE DE DIALYSE, UTILISATION D'UN PROCÉDÉ DE DOSAGE DU LIQUIDE DE DIALYSE

(30) Priorität: 13.12.2023 DE 102023134920
(43) Veröffentlichungstag der Anmeldung: 18.06.2025
(73) Patentinhaber: B. Braun Avitum AG, 34212 Melsungen (DE)
(72) Erfinder: Mueglich, Nicolas David, 34233 Fuldatal (DE)
(74) Vertreter: Patentanwälte Ruff, Wilhelm, Beier, Dauster & Partner mbB

(56) Entgegenhaltungen:
- EP-A1- 2 767 296
- EP-B1- 0 597 817
- DE-A1- 102021 129 003

## Beschreibung

Die Erfindung betrifft ein Verfahren zum Bestimmen einer Natrium- und Bicarbonationenkonzentration einer Dialysierflüssigkeit, die Verwendung eines Verfahrens zur Proportionierung der Dialysierflüssigkeit sowie die Verwendung einer Vorrichtung zur extrakorporalen Blutbehandlung zur Durchführung eines Verfahrens zum Bestimmen einer Natrium- und Bicarbonationenkonzentration einer Dialysierflüssigkeit.

Bei der extrakorporalen Blutbehandlung, beispielsweise Hämodialyse, wird das Blut eines Patienten durch einen Dialysator geleitet, durch den zur gleichen Zeit eine Dialysierflüssigkeit geleitet wird. Im Dialysator werden Blut und Dialysierflüssigkeit über eine semipermeable Membran in Kontakt gebracht, so dass ein Stoffaustausch zwischen dem Patientenblut und der Dialysierflüssigkeit stattfinden kann. Ziel einer derartigen Dialysebehandlung ist es, bei einem niereninsuffizienten Patienten das Blut zu entgiften und überschüssiges Wasser aus dem Körper zu entfernen.

Die Dialysierflüssigkeit besteht aus hochreinem Wasser, einem basischen Fluid, auch als basisches Konzentrat oder Bicarbonatkonzentrat bezeichnet, sowie einem sauren Fluid, auch als saures Konzentrat bezeichnet. Das basische Fluid bzw. basische Konzentrat enthält üblicherweise Natriumhydrogencarbonat (NaHCO₃). Das saure Fluid bzw. saure Konzentrat enthält bevorzugt Natriumchlorid (NaCl), Kaliumchlorid (KCI), Magnesiumchlorid (MgCl₂), Calciumchlorid (CaCl₂), Glucose und eine Säure wie Essigsäure (CH₃COOH).

In der Regel werden zur Herstellung oder Proportionierung der Dialysierflüssigkeit Dosierpumpen und Leitfähigkeitssonden eingesetzt. Eine Sonde misst dabei die Leitfähigkeit nach Zugabe des basischen Fluids zu hochreinem Wasser, das auch als Osmosewasser bezeichnet werden kann, mittels einer ersten Dosierpumpe. Eine weitere Sonde erfasst die Leitfähigkeit der gesamten Dialysierflüssigkeit, nachdem auch das saure Fluid mittels einer weiteren Dosierpumpe zugegeben worden ist. Dabei werden die Zugabemengen von basischem Fluid und saurem Fluid anhand der gemessenen Leitfähigkeiten reguliert (sogenannte leitfähigkeitsgesteuerte Proportionierung).

Die richtige Zusammensetzung der Dialysierflüssigkeit ist für das Wohlbefinden und die Lebenserwartung eines Patienten von großer Bedeutung. Dies gilt insbesondere in Bezug auf die Natrium- sowie Bicarbonationenkonzentration. Eine Messung der vorgenannten Konzentrationen findet in den Vorrichtungen zur extrakorporalen Blutbehandlung in der Regel indirekt über die Leitfähigkeit statt. Die Messung basiert auf einem linearen Modell, welches den Zusammenhang zwischen Leitfähigkeit und Konzentration approximiert. Hierfür werden Umrechnungsfaktoren benötigt, die für das basische Fluid bzw. saure Fluid spezifisch sind (konzentratspezifische Umrechnungsfaktoren). Diese Faktoren müssen experimentell für das jeweils verwendete basische Fluid bzw. saure Fluid bestimmt werden, indem für eine definierte Leitfähigkeit die Zusammensetzung des jeweiligen basischen Fluids bzw. sauren Fluids im Labor bestimmt wird. Durch Einsetzen der gemessenen Leitfähigkeit und der experimentell bestimmten Konzentrationen können die Umrechnungsfaktoren berechnet werden. Diese Methode ist jedoch mit diversen Nachteilen behaftet. So müssen die entsprechenden Laboruntersuchungen für jedes basische Fluid bzw. saure Fluid lokal in einem Dialysezentrum durchgeführt werden. Dies verursacht Kosten in nicht unerheblichem Ausmaß. Weiter hängt die Qualität der zu bestimmenden Umrechnungsfaktoren von der Qualität der jeweiligen Laboruntersuchung ab. Messfehler bei der Bestimmung der Konzentrationen führen zu einem systematischen Fehler in der Zusammensetzung der Dialysierflüssigkeit. Ein weiteres Problem stellt die Instabilität der zur Konzentrationsbestimmung benötigten Proben dar. Durch Entweichen von Kohlendioxid (CO₂) in die Luft wird die Bicarbonationenkonzentration mit der Zeit verfälscht. Außerdem fällt unlösliches Calciumcarbonat (CaCO₃) aus, was ebenfalls zu einer Verfälschung der Bicarbonationenkonzentration beiträgt. Schließlich stellt die Berechnung der Umrechnungsfaktoren eine komplexe und mithin zeitintensive Angelegenheit dar, die zudem einen erhöhten Schulungsaufwand auf Seiten des auszuführenden Personals erfordert.

Aus der EP 2 494 998 B1 ist ein Dialysatkonzentrationsüberwachungsverfahren bekannt, bei dem die Leitfähigkeit auf Basis eines Mischungsverhältnisses proportional verschoben wird. Das Verfahren arbeitet ausschließlich relativ. Liegt daher ein anfänglicher Fehler oder eine anfängliche Ungenauigkeit in der Zusammensetzung vor, so wird dieser Fehler bei jeder Änderung des Mischungsverhältnisses weitergegeben.

Weiterer relevanter Stand der Technik ist z.B. in den Dokumenten DE102021129003 A1, EP0597817 B1 und EP2767296 A1 offenbart.

### AUFGABE UND LÖSUNG

Der vorliegenden Erfindung liegt die Aufgabe zugrunde, ein Verfahren zum Bestimmen einer Natrium- und Bicarbonationenkonzentration einer Dialysierflüssigkeit bereitzustellen, das aus dem Stand der Technik bekannte Nachteile wenigstens teilweise umgeht. Weiter liegt der Erfindung die Aufgabe zugrunde, die Verwendung eines Verfahrens zur Herstellung oder Proportionierung einer Dialysierflüssigkeit sowie die Verwendung einer Vorrichtung zur extrakorporalen Blutbehandlung zur Durchführung eines Verfahrens zum Bestimmen einer Natrium- und Bicarbonationenkonzentration einer Dialysierflüssigkeit bereitzustellen.

Diese Aufgaben werden erfindungsgemäß gelöst durch ein Verfahren gemäß unabhängigem Anspruch 1, die Verwendung eines Verfahrens gemäß Anspruch 17 sowie die Verwendung einer Vorrichtung zur extrakorporalen Blutbehandlung gemäß Anspruch 18. Bevorzugte Ausgestaltungen der Erfindung sind Gegenstand der abhängigen Ansprüche. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung ein Verfahren zum Bestimmen einer Natriumionenkonzentration und Bicarbonationenkonzentration einer Dialysierflüssigkeit, insbesondere für die Hämodialyse und/oder Peritonealdialyse.

Das Verfahren weist, insbesondere in zeitlicher Reihenfolge oder nicht in zeitlicher Reihenfolge, die folgenden Schritte auf:
a) Bereitstellen eines verdünnten basischen Fluids durch Mischen eines basischen Fluids, das eine, insbesondere definierte, d.h. vorgegebene oder bekannte, oder nicht definierte, Bicarbonationenkonzentration (Hydrogencarbonationenkonzentration) aufweist, mit Wasser, d.h. hochreinem Wasser oder Osmosewasser, gemäß einem definierten, d.h. vorgegebenen oder bekannten, Mischungsverhältnis und Bereitstellen eines verdünnten sauren Fluids durch Mischen eines sauren Fluids, das eine, insbesondere definierte, d.h. vorgegebene oder bekannte, oder nicht definierte, Natriumionenkonzentration sowie eine, insbesondere definierte, d.h. vorgegebene oder bekannte, oder nicht definierte, Säurekonzentration aufweist, mit Wasser, d.h. hochreinem Wasser oder Osmosewasser, gemäß einem definierten, d.h. vorgegebenen oder bekannten, Mischungsverhältnis,
b) Herstellen einer Vorstufe der Dialysierflüssigkeit durch, insbesondere kontinuierliche oder diskontinuierliche, Zugabe des verdünnten basischen Fluids zu Wasser, d.h. hochreinem Wasser oder Osmosewasser,
c) Bestimmen eines Sollwerts für eine Bicarbonatleitfähigkeit der Dialysierflüssigkeit und/oder Vorstufe der Dialysierflüssigkeit auf Basis einer, insbesondere definierten, d.h. vorgegebenen oder bekannten, oder nicht definierten, Bicarbonationenkonzentration für die Dialysierflüssigkeit und/oder Vorstufe der Dialysierflüssigkeit und eines Parameters zur Umrechnung einer Bicarbonationenkonzentration in eine Bicarbonatleitfähigkeit oder umgekehrt, d.h. zur Umrechnung einer Bicarbonatleitfähigkeit in eine Bicarbonationenkonzentration, und Bestimmen eines Sollwerts für eine Leitfähigkeit, insbesondere End- oder Gesamtleitfähigkeit, der Dialysierflüssigkeit auf Basis einer, insbesondere definierten, d.h. vorgegebenen oder bekannten, oder nicht definierten, Natriumionenkonzentration für die Dialysierflüssigkeit und eines Parameters zur Umrechnung einer Natriumionenkonzentration in eine Leitfähigkeit, insbesondere End- oder Gesamtleitfähigkeit, der Dialysierflüssigkeit oder umgekehrt, d.h. zur Umrechnung einer Leitfähigkeit, insbesondere End- oder Gesamtleitfähigkeit, der Dialysierflüssigkeit in eine Natriumionenkonzentration,
d) Messen einer Leitfähigkeit der Vorstufe der Dialysierflüssigkeit,
e) Abgleichen der gemäß Schritt d) gemessenen Leitfähigkeit der Vorstufe der Dialysierflüssigkeit mit dem gemäß Schritt c) bestimmten Sollwert für die Bicarbonatleitfähigkeit der Dialysierflüssigkeit und/oder Vorstufe der Dialysierflüssigkeit und gegebenenfalls weitere, insbesondere kontinuierliche oder diskontinuierliche, Zugabe des verdünnten basischen Fluids, insbesondere mittels einer Förderpumpe, zu der Vorstufe der Dialysierflüssigkeit, bis der Sollwert für die Bicarbonatleitfähigleit der Dialysierflüssigkeit und/oder Vorstufe der Dialysierflüssigkeit erreicht oder im Wesentlichen erreicht ist (sogenannter Closed-Loop-Controller),
f) Herstellen der Dialysierflüssigkeit durch, insbesondere kontinuierliche oder diskontinuierliche, Zugabe des verdünnten sauren Fluids zu der Vorstufe der Dialysierflüssigkeit,
g) Messen einer Leitfähigkeit, insbesondere End- oder Gesamtleitfähigkeit, der Dialysierflüssigkeit,
h) Abgleichen der gemäß Schritt g) gemessenen Leitfähigkeit der Dialysierflüssigkeit mit dem gemäß Schritt c) bestimmten Sollwert für die Leitfähigkeit der Dialysierflüssigkeit und gegebenenfalls weitere, insbesondere kontinuierliche oder diskontinuierliche, Zugabe des verdünnten sauren Fluids, insbesondere mittels einer weiteren Förderpumpe, zu der Dialysierflüssigkeit, bis der Sollwert für die Leitfähigkeit der Dialysierflüssigkeit erreicht oder im Wesentlichen erreicht ist (sogenannter Closed-Loop-Controller), und
i) Bestimmen der Bicarbonationenkonzentration der Dialysierflüssigkeit basierend auf der gemäß Schritt d) gemessenen Leitfähigkeit der Vorstufe der Dialysierflüssigkeit und des Parameters zur Umrechnung einer Bicarbonationenkonzentration in eine Bicarbonatleitfähigkeit oder umgekehrt, d.h. zur Umrechnung einer Bicarbonatleitfähigkeit in eine Bicarbonationenkonzentration, sowie Bestimmen der Natriumionenkonzentration der Dialysierflüssigkeit basierend auf der gemäß Schritt g) gemessenen Leitfähigkeit der Dialysierflüssigkeit und des Parameters zur Umrechnung einer Natriumionenkonzentration in eine Leitfähigkeit der Dialysierflüssigkeit oder umgekehrt, d.h. zur Umrechnung einer Leitfähigkeit der Dialysierflüssigkeit in eine Natriumionenkonzentration,
wobei der Parameter zur Umrechnung einer Bicarbonationenkonzentration in eine Bicarbonatleitfähigkeit oder umgekehrt, d.h. zur Umrechnung einer Bicarbonatleitfähigkeit in eine Bicarbonationenkonzentration, und der Parameter zur Umrechnung einer Natriumionenkonzentration in eine Leitfähigkeit der Dialysierflüssigkeit oder umgekehrt, d.h. zur Umrechnung einer Leitfähigkeit der Dialysierflüssigkeit in eine Natriumionenkonzentration, jeweils automatisiert ermittelt werden.

Der Parameter zur Umrechnung einer Bicarbonationenkonzentration in eineBicarbonatleitfähigkeit oder umgekehrt kann im Sinne der vorliegenden Erfindung auch als Umrechnungsfaktor zur Umrechnung einer Bicarbonationenkonzentration in eineBicarbonatleitfähigkeit oder umgekehrt bezeichnet werden.

Der Parameter zur Umrechnung einer Natriumionenkonzentration in eine Leitfähigkeit der Dialysierflüssigkeit oder umgekehrt kann im Sinne der vorliegenden Erfindung auch als Umrechnungsfaktor zur Umrechnung einer Natriumionenkonzentration in eine Leitfähigkeit der Dialysierflüssigkeit oder umgekehrt bezeichnet werden.

Unter dem Ausdruck "Dialysierflüssigkeit" soll im Sinne der vorliegenden Erfindung eine, insbesondere gebrauchsfertige oder nicht gebrauchsfertige, Flüssigkeit für die Dialyse, insbesondere Hämodialyse und/oder Peritonealdialyse, verstanden werden, die sich aus Wasser, d.h. hochreinem Wasser (auch als Osmosewasser bezeichnet), dem basischen Fluid (auch als basisches Konzentrat bezeichnet) und dem sauren Fluid (auch als saures Konzentrat bezeichnet) zusammensetzt.

Unter dem Ausdruck "basisches Fluid" soll im Sinne der vorliegenden Erfindung ein unverdünntes basisches Fluid, d.h. ein unverdünntes Bicarbonatkonzentrat oder Bicarbonationenkonzentrat, verstanden werden, das eine definierte Bicarbonationenkonzentration aufweist.

Unter dem Ausdruck "verdünntes basisches Fluid" soll im Sinne der vorliegenden Erfindung ein basisches Fluid verstanden werden, welches, insbesondere in Bezug auf seine Konzentrationen, durch Mischen mit Wasser, d.h. hochreinem Wasser oder Osmosewasser, gemäß einem definierten, d.h. vorgegebenen oder bekannten, Mischungsverhältnis verdünnt ist oder einem verdünnten Zustand vorliegt.

Unter dem Ausdruck "saures Fluid" soll im Sinne der vorliegenden Erfindung ein unverdünntes saures Fluid, d.h. ein unverdünntes säurehaltige Konzentrat oder Säurekonzentrat, verstanden werden, das eine definierte Natriumionenkonzentration, insbesondere eine definierte Natriumionenkonzentration, Kaliumionenkonzentration, Magnesiumionenkonzentration und Calciumionenkonzentration sowie eine definierte Säurekonzentration, insbesondere Essigsäurekonzentration, aufweist.

Unter dem Ausdruck "verdünntes saures Fluid" soll im Sinne der vorliegenden Erfindung ein saures Fluid verstanden werden, welches, insbesondere in Bezug auf seine Konzentrationen, durch Mischen mit Wasser, d.h. hochreinem Wasser oder Osmosewasser, gemäß einem definierten, d.h. vorgegebenen oder bekannten, Mischungsverhältnis verdünnt ist oder einem verdünnten Zustand vorliegt.

Mit dem Ausdruck "Leitfähigkeit" ist im Sinne der vorliegenden Erfindung eine elektrische Leitfähigkeit gemeint.

Unter dem Ausdruck "Bicarbonatleitfähigkeit soll im Sinne der vorliegenden Erfindung eine auf Bicarbonationen und Natriumionen, insbesondere im Verhältnis 1 : 1, zurückgehende Leitfähigkeit verstanden werden.

Unter dem Ausdruck "End- oder Gesamtleitfähigkeit der Dialysierflüssigkeit" soll im Sinne der vorliegenden Erfindung eine auf alle in der Dialysierflüssigkeit enthaltene lonenspezies, insbesondere Kationenspezies, bevorzugt eine auf, insbesondere, die Natriumionen sowie vorzugsweise Kaliumionen, Magnesiumionen und Calciumionen, zurückgehende Leitfähigkeit verstanden werden.

Bei den gemäß den Schritten d) und g) gemessenen Leitfähigkeiten kann es sich insbesondere jeweils um eine temperaturkompensierte Leitfähigkeit handeln.

Unter dem Ausdruck "temperaturkompensierte Leitfähigkeit" soll im Sinne der vorliegenden Erfindung eine Leitfähigkeit verstanden werden, die der Leitfähigkeit bei einer Raum- oder Umgebungstemperatur von 25 °C entspricht.

Der im Zusammenhang von Schritt e) verwendete Ausdruck "im Wesentlichen erreicht" bedeutet im Sinne der vorliegenden Erfindung, dass die gemäß Schritt d) gemessene Leitfähigkeit der Vorstufe der Dialysierflüssigkeit ≤ 1 %, insbesondere < 1 %, von dem Sollwert für die Bicarbonatleitfähigkeit der Dialysierflüssigkeit und/oder Vorstufe der Dialysierflüssigkeit abweichen kann.

Der im Zusammenhang von Schritt h) verwendete Ausdruck "im Wesentlichen erreicht" bedeutet im Sinne der vorliegenden Erfindung, dass die gemäß Schritt g) gemessene Leitfähigkeit der Dialysierflüssigkeit ≤ 1 %, insbesondere < 1 %, von dem Sollwert für die Leitfähigkeit der Dialysierflüssigkeit abweichen kann.

Der Schritt c) kann zeitlich insbesondere vor oder nach Schritt a) oder Schritt b), insbesondere zwischen den Schritten a) und b) oder, bevorzugt, zwischen den Schritten b) und d) durchgeführt werden.

Unter dem Ausdruck "hochreines Wasser" oder Osmosewasser soll im Sinne der vorliegenden Erfindung keimfreies Wasser ohne Elektrolyte oder ohne nennenswerte **Elektrolyte,** d.h. mit einem Elektrolytanteil von maximal 0.01 mEq/l, verstanden werden. Weiter kann das hochreine Wasser bzw. Osmosewasser insbesondere eine Leitfähigkeit ≤ 1.1 µS/cm bei 20 °C und/oder eine Konzentration bakterieller Endotoxine < 0,25 IE/ml (ca. 25 ng/l) und/oder eine Konzentration von gesamtem organischen Kohlenstoff (TOC) ≤ 0,5 mg/l und/oder eine Nitratkonzentration ≤ 0,2 mg/l aufweisen.

Durch das automatisierte Ermitteln von Parametern zur Umrechnung von Leitfähigkeiten in Natrium- sowie Bicarbonationenkonzentrationen oder umgekehrt können die eingangs im Zusammenhang herkömmlicher Verfahren genannten Nachteile minimiert oder vermieden werden. Kosten- und zeitintensive sowie fehleranfällige Laboruntersuchungen und Anschlussberechnungen sind somit entbehrlich. Dies führt zu einer signifikanten Vereinfachung der Vorbereitung sowie Durchführung einer extrakorporalen Blutbehandlung und insbesondere zu einer Erhöhung der Sicherheit für zu behandelnde Patienten.

In Ausgestaltung der Erfindung weist das basische Fluid ferner eine, insbesondere definierte, d.h. vorgegebene oder bekannte, oder nicht definierte, Natriumionenkonzentration auf.

Insbesondere wird das basische Fluid in Form einer wässrigen basischen Lösung bereitgestellt. Vorzugsweise wird das basische Fluid in Form einer wässrigen basischen Lösung bereitgestellt, die Natriumbicarbonat, auch als Natriumhydrogencarbonat bezeichnet, (NaHCO₃) aufweist. Eine solche Lösung kann im Sinne der vorliegenden Erfindung auch als wässrige basische Natriumbicarbonat-Lösung bzw. Natriumhydrogencarbonat-Lösung bezeichnet werden.

Das basische Fluid kann eine Bicarbonationenkonzentration von 600 mmol/I bis 1500 mmol/l, insbesondere 1000 mmol/I bis 1500 mmol/l, aufweisen. Beispielsweise kann das basische Fluid eine Bicarbonationenkonzentration von 1000 mmol/I oder 1300 mmol/I aufweisen.

Weiter kann das basische Fluid eine Natriumionenkonzentration von 600 mmol/I bis 1500 mmol/l, insbesondere 1000 mmol/I bis 1500 mmol/l, aufweisen. Beispielsweise kann das basische Fluid eine Natriumionenkonzentration von 1000 mmol/I oder 1300 mmol/I aufweisen.

Das saure Fluid kann ferner wenigstens eine weitere, insbesondere definierte, d.h. vorgegebene oder bekannte, oder nicht definierte, Kationenspezieskonzentration aufweisen, insbesondere ausgewählt aus der Gruppe bestehend aus eine, insbesondere definierte, d.h. vorgegebene oder bekannte, oder nicht definierte, Kaliumionenkonzentration, eine, insbesondere definierte, d.h. vorgegebene oder bekannte, oder nicht definierte, Magnesiumionenkonzentration, eine, insbesondere definierte, d.h. vorgegebene oder bekannte, oder nicht definierte, Calciumionenkonzentration und Kombinationen von wenigstens zwei der vorgenannten Kationenspezieskonzentrationen.

In weiterer Ausgestaltung der Erfindung weist das saure Fluid ferner eine, insbesondere definierte, d.h. vorgegebene oder bekannte, oder nicht definierte, Kaliumionenkonzentration, eine, insbesondere definierte, d.h. vorgegebene oder bekannte, oder nicht definierte, Magnesiumionenkonzentration und eine, insbesondere definierte, d.h. vorgegebene oder bekannte, oder nicht definierte, Calciumionenkonzentration auf.

Bei der Säure des sauren Fluids kann es sich grundsätzlich um eine anorganische Säure und/oder organische Säure handeln. Die Säure kann insbesondere ausgewählt sein aus der Gruppe bestehend aus Salzsäure, Essigsäure, Milchsäure, Acetessigsäure, Zitronensäure, Äpfelsäure, Maleinsäure, Brenztraubensäure, Bernsteinsäure und Kombinationen, insbesondere Mischungen, von wenigstens zwei der vorgenannten Säuren.

Bevorzugt weist das saure Fluid Essigsäure als Säure auf.

Weiter kann das saure Fluid ferner eine osmotisch wirksame Verbindung aufweisen. Die osmotisch wirksame Verbindung kann ausgewählt sein aus der Gruppe bestehend aus Glycin, Monosaccharide, Disaccharide, Polysaccharide, Zuckeralkohole, Gelatine, Aminosäuren und Kombinationen, insbesondere Mischungen, von wenigstens zwei der vorgenannten osmotisch wirksamen Verbindungen. Die Monosaccharide können ausgewählt sein aus der Gruppe bestehend aus Glucose, Fructose, Galactose und Kombinationen, insbesondere Mischungen, von wenigstens zwei der vorgenannten Monosaccharide. Die Disaccharide können ausgewählt sein aus der Gruppe bestehend aus Saccharose, Maltose, Trehalose und Kombinationen, insbesondere Mischungen, von wenigstens zwei der vorgenannten Disaccharide. Die Polysaccharide können ausgewählt sein aus der Gruppe bestehend aus Dextrin, Stärke, Polyglucose, Hydroxyethylstärke und Kombinationen, insbesondere Mischungen, von wenigstens zwei der vorgenannten Polysaccharide. Die Zuckeralkohole können ausgewählt sein aus der Gruppe bestehend aus Xylit, Mannit, Sorbit und Kombinationen, insbesondere Mischungen, von wenigstens zwei der vorgenannten Zuckeralkohole. Bei den Aminosäuren kann es sich grundsätzlich um essentielle Aminosäuren und/oder nichtessentielle Aminosäuren handeln.

Bevorzugt weist das saure Fluid Glucose als osmotisch wirksame Verbindung auf.

Insbesondere wird das saure Fluid in Form einer wässrigen sauren Lösung bereitgestellt. Vorzugsweise wird das saure Fluid in Form einer wässrigen sauren Lösung bereitgestellt, die Natriumchlorid, Kaliumchlorid, Magnesiumchlorid, Calciumchlorid und eine Säure, insbesondere Essigsäure oder Zitronensäure, aufweist.

Besonders bevorzugt wird das saure Fluid in Form einer wässrigen sauren Lösung bereitgestellt, die Natriumchlorid, Kaliumchlorid, Magnesiumchlorid, Calciumchlorid, eine osmotisch wirksame Verbindung, insbesondere Glucose, und eine Säure, insbesondere Essigsäure oder Zitronensäure, aufweist. Bezüglich weiterer in Frage kommender osmotisch wirksamer Verbindungen sowie weiterer in Frage kommender Säuren wird vollumfänglich auf die bisherigen Ausführungen Bezug genommen.

Das saure Fluid kann eine Natriumionenkonzentration von 95 mmol/I bis 110 mmol/l, insbesondere 100 mmol/I bis 103 mmol/l, aufweisen.

Weiter kann das saure Fluid eine Kaliumionenkonzentration von > 0 mmol/I bis 5 mmol/l, insbesondere 1 mmol/I bis 4 mmol/l, aufweisen.

Weiter kann das saure Fluid eine Magnesiumionenkonzentration von > 0 mmol/I bis 1,5 mmol/l, insbesondere 0,5 mmol/l, aufweisen.

Weiter kann das saure Fluid eine Calciumionenkonzentration von > 0 mmol/I bis 3 mmol/l, insbesondere 1 mmol/I bis 1,5 mmol/l, aufweisen.

Die Schritte d) und g) werden bevorzugt mittels Leitfähigkeitssonden oder -elektroden oder anderen geeigneten Leitfähigkeitsmesseinrichtungen oder -geräten durchgeführt.

Beim Durchführen von Schritt a) können/kann das basische Fluid mit dem Wasser gemäß einem definierten Mischungsverhältnis von dem basischem Fluid zu dem Wasser von 1 : 30 oder 1 : 36 gemischt und/oder das saure Fluid mit dem Wasser gemäß einem definierten Mischungsverhältnis von dem saurem Fluid zu dem Wasser von 1 : 34 oder 1 : 44 gemischt werden. Entsprechende Mischungsverhältnisse können vorteilhafterweise Etiketten oder Labels von Behältern, die das basische Fluid oder saure Fluid enthalten, entnommen werden. Vorteilhaft ist ferner, dass alle für die extrakorporale Blutbehandlung verwendbaren Säurearten berücksichtigt werden können.

In weiterer Ausgestaltung der Erfindung wird der Sollwert für die Bicarbonatleitfähigkeit der Dialysierflüssigkeit und/oder Vorstufe der Dialysierflüssigkeit mithilfe eines linearen Modells, insbesondere gemäß folgender Gleichung (1) bestimmt: $BICLF = {c}_{bic} \left(mmol/l\right) \times {Λ}_{m , bic}$ (1) wobei
- BICLF: dem Sollwert für die Bicarbonatleitfähigkeit der Dialysierflüssigkeit und/oder Vorstufe der Dialysierflüssigkeit,
- c_{bic}: der Bicarbonationenkonzentration für die Dialysierflüssigkeit und/oder Vorstufe der Dialysierflüssigkeit und
- Λ*_{m,bic}*: dem Parameter zur Umrechnung einer Bicarbonationenkonzentration in eine Bicarbonatleitfähigkeit oder umgekehrt entspricht.

In weiterer Ausgestaltung der Erfindung wird der Sollwert für die Leitfähigkeit der Dialysierflüssigkeit mithilfe eines linearen Modells, insbesondere gemäß folgender Gleichung (2) bestimmt: $ENDLF = \left\{\left[{c}_{Na + , gesamt}\left(mmol\left(l\right)-{c}_{bic}\left(mmol/l\right)\right.\right]\times {Λ}_{m , acid}\right\} + \left[{c}_{bic}\left(mmol/l\right)\times {Λ}_{m , bic}\right]$ (2) wobei
- ENDLF: dem Sollwert für die Leitfähigkeit der Dialysierflüssigkeit,
- c_{Na+,gesamt}: der Natriumionenkonzentration für die Dialysierflüssigkeit,
- c_{bic}: der Bicarbonationenkonzentration für die Dialysierflüssigkeit und/oder Vorstufe der Dialysierflüssigkeit,
- Λ*_{m,acid}*: dem Parameter zur Umrechnung einer Natriumionenkonzentration in eine Leitfähigkeit der Dialysierflüssigkeit oder umgekehrt und
- Λ*_{m,bic}*: dem Parameter zur Umrechnung einer Bicarbonationenkonzentration in eine Bicarbonatleitfähigkeit oder umgekehrt entspricht.

In weiterer Ausgestaltung der Erfindung werden der Parameter zur Umrechnung einer Bicarbonationenkonzentration in eine Bicarbonatleitfähigkeit oder umgekehrt sowie der Parameter zur Umrechnung einer Natriumionenkonzentration in eine Leitfähigkeit der Dialysierflüssigkeit oder umgekehrt auf Basis von Konzentrationen des verdünnten basischen Fluids und/oder des verdünnten sauren Fluids, bevorzugt, insbesondere ausschließlich, des verdünnten sauren Fluids, automatisiert ermittelt. Dies hat den Vorteil, dass das Verfahren hinsichtlich des basischen Fluids und/oder sauren Fluids, bevorzugt des sauren Fluids, keinerlei Beschränkungen unterliegt. Das Verfahren ist somit grundsätzlich mit allen kommerziell erhältlichen basischen Fluiden sowie sauren Fluiden durchführbar.

In weiterer Ausgestaltung der Erfindung wird der Parameter zur Umrechnung einer Bicarbonationenkonzentration in eine Bicarbonatleitfähigkeit oder umgekehrt auf Basis einer Bicarbonationenkonzentration des verdünnten basischen Fluids und einer Säurekonzentration des verdünnten sauren Fluids automatisiert ermittelt.

In weiterer Ausgestaltung der Erfindung wird der Parameter zur Umrechnung einer Natriumionenkonzentration in eine Leitfähigkeit der Dialysierflüssigkeit oder umgekehrt auf Basis einer Bicarbonationenkonzentration des verdünnten basischen Fluids, einer Natriumionenkonzentration des verdünnten basischen Fluids, einer Natriumionenkonzentration des verdünnten sauren Fluids, einer Kaliumionenkonzentration des verdünnten sauren Fluids, einer Magnesiumionenkonzentration des verdünnten sauren Fluids, einer Calciumionenkonzentration des verdünnten sauren Fluids und einer Säurekonzentration des verdünnten sauren Fluids automatisiert ermittelt.

In weiterer Ausgestaltung der Erfindung wird der Parameter zur Umrechnung einer Bicarbonationenkonzentration in eine Bicarbonatleitfähigkeit oder umgekehrt gemäß folgender Gleichung (3) bestimmt: ${Λ}_{m , bic} = 0.0852 \frac{mS l}{mmol cm} \frac{{c}_{bic , 0} + {c}_{acid , 0} \left[\frac{mEq}{l}\right]}{{c}_{bic , 0}}$ (3) wobei
- Λ*_{m,bic}*: dem Parameter zur Umrechnung einer Bicarbonationenkonzentration in eine Bicarbonatleitfähigkeit oder umgekehrt,
- *c*_{*bic,*0}: einer Bicarbonationenkonzentration des verdünnten basischen Fluids und
- *c*_{*acid,*0}: einer Säurekonzentration des verdünnten sauren Fluids entspricht.

Obiger Ausgestaltung der Erfindung liegen die nachfolgenden Annahmen/Überlegungen zugrunde:
Wird das verdünnte basische Fluid in Form einer wässrigen basischen Natriumbicarbonat-Lösung bereitgestellt, weist das verdünnte basische Fluid, insbesondere im Wesentlichen, zwei Typen geladener Bestandteile auf, nämlich Natriumionen sowie Bicarbonationen. Diese Bestandteile sind einfach geladen und tragen daher zur Leitfähigkeit der Vorstufe der Dialysierflüssigkeit mit bei. Wird der Zusammenhang zwischen Leitfähigkeit und Konzentration mit einem linearen Modell approximiert, bedeutet dies, dass jeder geladene Bestandteil des verdünnten basischen Fluids in der Vorstufe der Dialysierflüssigkeit den gleichen Anteil zur Leitfähigkeit beiträgt. Ein Parameter zur Umrechnung der Leitfähigkeit in die Bicarbonationenkonzentration beschreibt dabei den Leitfähigkeitsbeitrag pro Natriumbicarbonat in einem definierten Volumenelement (mS/cm)/(mmol/I).

Für die in der extrakorporalen Blutbehandlung, insbesondere Dialyse, üblichen Bicarbonationenkonzentrationen von, insbesondere circa, 35 mmol/I lässt sich der Parameter zur Umrechnung der Leitfähigkeit in die Bicarbonationenkonzentration experimentell zu 0,083 (mS/cm)/(mmol/l) bestimmen. Dabei muss jedoch berücksichtigt werden, dass die Bicarbonationenkonzentration der Vorstufe der Dialysierflüssigkeit nicht der Bicarbonationenkonzentration der Dialysierflüssigkeit entspricht. Dies hat vor allem zwei Ursachen:
Erstens wird die Vorstufe der Dialysierflüssigkeit durch die Zugabe des verdünnten sauren Fluids verdünnt. Der exakte Grad der Verdünnung hängt insbesondere davon ab, ob ein 1+34 oder ein 1+44 saures Fluid verwendet wird. Dabei bedeutet der Ausdruck "1+34 saures Fluid" im Sinne der vorliegenden Erfindung, dass 1 Teil des sauren Fluids und 34 Teile Wasser, d.h. hochreines Wasser oder Osmosewasser, gemischt werden, um ein entsprechend verdünntes saures Fluid zu erhalten. Der Ausdruck "1+44 saures Fluid" bedeutet dementsprechend im Sinne der vorliegenden Erfindung, dass 1 Teil des sauren Fluids und 44 Teile Wasser, d.h. hochreines Wasser oder Osmosewasser, gemischt werden, um ein entsprechend verdünntes saures Fluid zu erhalten. Weiter ist der Anteil des verdünnten sauren Fluids auch von der auf einer Vorrichtung zur extrakorporalen Blutbehandlung eingestellten Natriumionenkonzentration abhängig. Die Vorrichtung wird im Allgemeinen bei größeren Natriumioneneinstellwerten mehr verdünntes saures Fluid hinzudosieren. In Summe wird die Bicarbonationenkonzentration zwischen 1,7 % und 3,3 % reduziert. Im Mittel wird die Bicarbonationenkonzentration um, insbesondere circa, 2,6 % reduziert. Deswegen wird zur Kompensation der Parameter zur Umrechnung der Leitfähigkeit in die Bicarbonationenkonzentration um 2,6 % auf 0,0852 (mS/cm)/(mmol/l) erhöht.

Zweitens weist das verdünnte saure Fluid eine Säure auf, die einen Teil der Bicarbonationen neutralisiert. Als Produkte entstehen Kohlensäure und ein Salz der Säure (beispielsweise Acetat oder Zitrat). Der Umrechnungsfaktor wird daher ein zweites Mal kompensiert, um dieser Neutralisation Rechnung zu tragen. Eine gebrauchsfertige Dialysierflüssigkeit enthält üblicherweise eine Bicarbonationenkonzentration von 32 mmol/I und 3 mmol Salz der Säure, insbesondere Acetat. Deswegen müssen 35 mmol/I Bicarbonationen bereitgestellt werden, da 3 mmol/I durch die Säure, insbesondere Essigsäure, neutralisiert werden. Der Umrechnungsfaktor wird kompensiert auf 0,0852 x 35/32 = 0,09319 (mS/cm)/(mmol/I).

Allgemein ergibt sich hieraus obige Gleichung (3).

In weiterer Ausgestaltung der Erfindung wird der Parameter zur Umrechnung einer Natriumionenkonzentration in eine Leitfähigkeit der Dialysierflüssigkeit oder umgekehrt gemäß folgender Gleichung (4) bestimmt: ${Λ}_{m , acid , kor} = \frac{11 \frac{mS}{cm}}{{c}_{Na , {acid}^{+}} \left(11\frac{mS}{cm}\right) + {c}_{acid} \left(11\frac{mS}{cm}\right)}$ (4) wobei
Λ*_{m,acid,kor}* dem Parameter zur Umrechnung einer Natriumionenkonzentration in eine Leitfähigkeit der Dialysierflüssigkeit oder umgekehrt,
${c}_{Na , {acid}^{+}} \left(11\frac{mS}{cm}\right)$ einer Natriumionenkonzentration des verdünnten sauren Fluids entspricht, die eine Leitfähigkeit des verdünnten sauren Fluids von 11 mS/cm bewirkt, und
${c}_{acid} \left(11\frac{mS}{cm}\right)$ einer Säurekonzentration des verdünnten sauren Fluids entspricht, dessen Natriumionenkonzentration eine Leitfähigkeit des verdünnten sauren Fluids von 11 mS/cm bewirkt.

In weiterer Ausgestaltung der Erfindung wird die Natriumionenkonzentration des verdünnten sauren Fluids, die eine Leitfähigkeit des verdünnten sauren Fluids von 11 mS/cm bewirkt, gemäß folgender Gleichung (5) bestimmt: ${c}_{Na , {acid}^{+}} \left(11\frac{mS}{cm}\right) = \frac{11 \frac{mS}{cm}}{{Λ}_{m , acid}}$ (5) wobei
${c}_{Na , {acid}^{+}} \left(11\frac{mS}{cm}\right)$ der Natriumionenkonzentration des verdünnten sauren Fluids entspricht, die eine Leitfähigkeit des verdünnten sauren Fluids von 11 mS/cm bewirkt, und
Λ*_{m,acid}* einem unkorrigierten Parameter zur Umrechnung einer Natriumionenkonzentration in eine Leitfähigkeit, insbesondere End- oder Gesamtleitfähigkeit, der Dialysierflüssigkeit oder umgekehrt, d.h. zur Umrechnung einer Leitfähigkeit, insbesondere End- oder Gesamtleitfähigkeit, der Dialysierflüssigkeit in eine Natriumionenkonzentration, entspricht.

In weiterer Ausgestaltung der Erfindung wird der unkorrigierter Parameter Λ*_{m,acid}* zur Umrechnung einer Natriumionenkonzentration in eine Leitfähigkeit der Dialysierflüssigkeit oder umgekehrt gemäß folgender Gleichung (6) bestimmt: ${Λ}_{m , acid} = 0.09921 \frac{mS l}{mmol cm} \frac{{c}_{{Na}^{+} , 0} + {c}_{{K}^{+} , 0} + 2 ∗ {c}_{{Mg}^{+} , 0} + 2 ∗ {c}_{{Ca}^{+} , 0}}{{c}_{{Na}^{+} , 0}}$ (6) wobei
- Λ*_{m,acid}*: **dem** unkorrigierten Parameter zur Umrechnung einer Natriumionenkonzentration in eine Leitfähigkeit der Dialysierflüssigkeit oder umgekehrt,
- *c*_{*Na*⁺*,*0}: einer Natriumionenkonzentration des verdünnten sauren Fluids,
- *c*_{*K*⁺*,*0}: einer Kaliumionenkonzentration des verdünnten sauren Fluids,
- 2 * *c*_{*Mg+,*0}: einer Magnesiumionenkonzentration des verdünnten sauren Fluidsund
- 2 * *c*_{*Ca*^{+,}0}: einer Calciumionenkonzentration des verdünnten sauren Fluids entspricht.

In weiterer Ausgestaltung der Erfindung wird die Säurekonzentration des verdünnten sauren Fluids, dessen Natriumionenkonzentration eine Leitfähigkeit des verdünnten sauren Fluids von 11 mS/cm bewirkt, gemäß folgender Gleichung (7) bestimmt: ${c}_{acid} \left(11\frac{mS}{cm}\right) = \frac{{c}_{acid , 0} \left[\frac{mEq}{l}\right] ∗ {c}_{Na , {acid}^{+}} \left(11\frac{mS}{cm}\right)}{{c}_{{Na}^{+} , 0} - {c}_{bic , 0} - {c}_{acid , 0} \left[\frac{mEq}{l}\right]}$ (7) wobei
${c}_{acid} \left(11\frac{mS}{cm}\right)$ einer Säurekonzentration des verdünnten sauren Fluids entspricht, dessen Natriumionenkonzentration zu einer Leitfähigkeit von 11 mS/cm führt,
*c*_{*bic,*0} einer Bicarbonationenkonzentration des verdünnten basischen Fluids entspricht,
*c*_{*acid,*0} einer Säurekonzentration des verdünnten sauren Fluids entspricht,
*c*_{*Na*⁺*,*0} einer Natriumionenkonzentration des verdünnten sauren Fluids entsprichtund
${c}_{Na , {acid}^{+}} \left(11\frac{mS}{cm}\right)$ einer Natriumionenkonzentration des verdünnten sauren Fluids entspricht, die eine Leitfähigkeit des verdünnten sauren Fluids von 11 mS/cm bewirkt.

Den vier vorhergehenden Ausgestaltungen der Erfindung liegen die folgenden Annahmen/Überlegungen zugrunde:
Das verdünnte saure Fluid weist bevorzugt Natrium-, Kalium-, Magnesium- und Calciumionen, insbesondere als Bestandteil von Natriumchlorid, Kaliumchlorid, Magnesiumchlorid und Calciumchlorid, auf. Mithin tragen die vorgenannten Komponenten zur Leitfähigkeit des verdünnten sauren Fluids bei.

Die Säurekonzentration des verdünnten sauren Fluids trägt prinzipiell kaum zur Leitfähigkeit bei, da es sich bei der Säure des verdünnten sauren Fluids in der Regel um eine Säure handelt, die in wässriger Lösung lediglich eine geringe Dissoziation erfährt. Allerdings wird das verdünnten saure Fluid zum Herstellen der Dialysierflüssigkeit zu der Bicarbonationen aufweisenden Vorstufe der Dialysierflüssigkeit hinzugegeben, wodurch es zu einer Säure-Basen-Reaktion kommt. Das entstehende Salz der Säure, beispielsweise ein Acetatsalz, trägt zur Leitfähigkeit bei. Dem entgegenstehend kommt es durch die Neutralisation der Bicarbonationen (durch Umsetzung mit der Säure) zur Bildung von nicht oder kaum zur Leitfähigkeit beitragender Kohlensäure. Netto wird hierdurch die Leitfähigkeit somit nicht oder kaum verändert, so dass dieser Beitrag vernachlässigt werden kann.

Da zum Zeitpunkt der Zugabe des verdünnten sauren Fluids zu der Vorstufe der Dialysierflüssigkeit das Bicarbonat bereits hinzugegeben ist, ergeben sich zwei Faktoren für die Gesamtleitfähigkeit: der Beitrag der Bicarbonationen, der zuvor bestimmt wurde, und der Beitrag des verdünnten sauren Fluids. Der Beitrag des verdünnten sauren Fluids wird ebenfalls über ein lineares Modell approximiert, bei welchem davon ausgegangen wird, dass jede zur Leitfähigkeit beitragende Komponente des verdünnten sauren Fluids den gleichen Beitrag zur Leitfähigkeit liefert. Dabei wird auf die gewünschte Natriumionenkonzentration normiert.

Grundsätzlich lässt sich der Leitfähigkeitsbeitrag des verdünnten sauren Fluids gemäß nachfolgender Gleichung bestimmen:
Leitfähigkeitsbeitrag des verdünnten sauren Fluids = Natriumionenkonzentration des verdünnten sauren Fluids x Parameter zur Umrechnung einer Natriumionenkonzentration in eine Leitfähigkeit der Dialysierflüssigkeit oder umgekehrt.

Bei dem verdünnten sauren Fluid trägt, wie vorstehend beschrieben, nicht nur das Natriumchlorid, sondern ggf. auch weitere Elektrolyte, vorzugsweise Kaliumchlorid, Magnesiumchlorid und Calciumchlorid, zur Leitfähigkeit bei. Der Leitfähigkeitsbeitrag des verdünnten sauren Fluids je gewünschtem Natriumion ist also umso höher, je größer der Anteil der anderen Elektrolyte ist. Daher ergeben sich prinzipiell unterschiedliche Parameter zur Umrechnung einer Natriumionenkonzentration in eine Leitfähigkeit der Dialysierflüssigkeit oder umgekehrt, je nach Anteilen der Elektrolyte.

Im Rahmen der vorliegenden Erfindung wird (vereinfachend) angenommen, dass der Leitfähigkeitsbeitrag jedes Elektrolyts identisch ist. Experimentell konnte bestimmt werden, dass der Leitfähigkeitsbeitrag von Natriumchlorid bei einer für die extrakorporale Blutbehandlung, insbesondere Dialyse, typischen Konzentration von, insbesondere circa, 140 mmol/I bei 0,09921 (mS/cm)/(mmol/l) liegt.

Die hier (bislang) beschriebene Methode definiert daher für das verdünnte saure Fluid einen Parameter zur Umrechnung der Konzentration in die Leitfähigkeit oder umgekehrt gemäß obiger Gleichung (6).

Für den vorgenannten Umrechnungsparameter ist jedoch ein weiterer Kompensationsfaktor notwendig. Dieser ergibt sich daraus, dass die Gesamtnatriumionenkonzentration aus zwei Beiträgen resultiert, nämlich dem Natriumionenbeitrag aus dem verdünnten sauren Fluid und dem Natriumionenbeitrag aus dem verdünnten basischen Fluid. Für den Natriumionenbeitrag aus dem verdünnten basischen Fluid geht die bisherige Methode davon aus, dass für jedes Bicarbonation ein Natriumion hinzugefügt wird. Dies ist jedoch nicht vollständig korrekt. Durch die oben erwähnte Neutralisation mit der Säure des verdünnten sauren Fluids wird der Bicarbonationenanteil in der Dialysierflüssigkeit reduziert. Dies wurde im Rahmen der vorliegenden Erfindung für den Parameter zur Umrechnung einer Bicarbonationenkonzentration in eine Bicarbonatleitfähigkeit oder umgekehrt durch eine Kompensationsrechnung berücksichtigt. Der Natriumionenanteil des verdünnten basischen Fluids ist aber durch die bei Zugabe des verdünnten sauren Fluids zu der Vorstufe der Dialysierflüssigkeit stattfindenden Neutralisation nicht betroffen. Letztlich ist der durch das verdünnten basische Fluid beigefügte Anteil an Natriumionen somit größer als der Bicarbonationenanteil. Dieser zusätzliche Natriumionenanteil kann berücksichtigt werden, indem der Parameter zur Umrechnung der Konzentration in die Leitfähigkeit oder umgekehrt für das verdünnten saure Fluid entsprechend modifiziert wird.

Dazu wird zunächst eine Natriumionenkonzentration berechnet, die - für sich genommen - zu einer Leitfähigkeit von 11 mS/cm führt. Dieser Leitfähigkeitswert entspricht typischerweise dem Beitrag des verdünnten sauren Fluids zur Leitfähigkeit einer Dialysierflüssigkeit mit einer Gesamtleitfähigkeit von 14 mS/cm, wovon eine Leitfähigkeit von 3 mS/cm auf die Bicarbonationen zurückgeht. Diese Natriumionenkonzentration kann gemäß obiger Gleichung (5) bestimmt werden:
Das proportionale Modell geht davon aus, dass ein Natriumion für ein Bicarbonation hinzugefügt wird. Für Bicarbonationen trifft dies jedoch nach der Reaktion mit der Säure des verdünnten sauren Fluids nicht zu. In Wirklichkeit wird für jedes Bicarbonation, das durch die Säure eliminiert wird, ein zusätzliches Natriumion hinzugefügt.

Daher wird in einem dritten Schritt die Säurekonzentration des verdünnten sauren Fluids, dessen Natriumionenkonzentration bei einer Dialysierflüssigkeit mit einer Gesamtleitfähigkeit von 14 mS/cm einen Leitfähigkeitsbeitrag von 11 mS/cm beisteuert, gemäß obiger Gleichung (7) ermittelt.

Der Parameter zur Umrechnung einer Natriumionenkonzentration in eine Leitfähigkeit der Dialysierflüssigkeit oder umgekehrt kann in einem vierten Schritt gemäß obiger Gleichung (4) berechnet werden, d.h. indem 11 mS/cm und die Summe der durch das verdünnte saure Fluid zugegebenen Natriumionen geteilt werden, korrigiert um die Menge an Natriumionen, die aufgrund der Säure-Base-Reaktion zwischen dem Bicarbonat des verdünnten basischen Fluids und der Säure des verdünnten sauren Fluids vernachlässigt wird.

Bevorzugt wird beim Durchführen von Schritt i) die Bicarbonationenkonzentration der Dialysierflüssigkeit mithilfe eines linearen Modells, insbesondere gemäß folgender Gleichung (8), bestimmt: ${{c}^{+}}_{bic} \left(mmol/l\right) = {BICLF}^{+} / {Λ}_{m , bic}$ (8) wobei
- c⁺_{bic}: der Bicarbonationenkonzentration der Dialysierflüssigkeit,
- BICLF⁺: der gemäß Schritt d) gemessenen Leitfähigkeit der Vorstufe der Dialysierflüssigkeit, und
- Λ*_{m,bic}*: dem Parameter zur Umrechnung einer Bicarbontationenkonzentration in eine Bicarbonatleitfähigkeit oder umgekehrt entspricht.

Weiter bevorzugt wird beim Durchführen von Schritt i) die Natriumionenkonzentration der Dialysierflüssigkeit mithilfe eines linearen Modells, insbesondere gemäß folgender Gleichung (9) bestimmt: ${{c}^{+}}_{{Na}^{+} , gesamt} \left(mmol/l\right) = \left\{{ENDLF}^{+}-\left[{{c}^{+}}_{bic}\left(mmol/l\right)\times {Λ}_{m , bic}\right]\right\} / {Λ}_{m , acid , kor} + {{c}^{+}}_{bic} \left(mmol/l\right.$ (9) wobei
- c⁺_{Na+,gesamt}: der Natriumionenkonzentration der Dialysierflüssigkeit,
- ENDLF⁺: der gemäß Schritt g) gemessenen Leitfähigkeit der Dialysierflüssigkeit,
- c⁺_{bic}: der Bicarbonationenkonzentration der Dialysierflüssigkeit,
- Λ*_{m,acid,kor}*: dem Parameter zur Umrechnung einer Natriumionenkonzentration in eine Leitfähigkeit der Dialysierflüssigkeit oder umgekehrt und
- Λ*_{m,bic}*: dem Parameter zur Umrechnung einer Bicarbontationenkonzentration in eine Bicarbonatleitfähigkeit oder umgekehrt entspricht.

Alternativ kann der Parameter zur Umrechnung einer Bicarbontationenkonzentration in eine Bicarbonatleitfähigkeit oder umgekehrt gemäß folgender Gleichung (3#) bestimmt werden: ${Λ}_{m , bic} = 0.0852 \frac{mS l}{mmol cm}$ (3#) wobei
- Λ*_{m,bic}*: dem Parameter zur Umrechnung einer Bicarbontationenkonzentration in eine Bicarbonaleitfähigkeit oder umgekehrt entspricht.

Der Parameter zur Umrechnung einer Bicarbontationenkonzentration in eine Bicarbonatleitfähigkeit oder umgekehrt ist in diesem Fall somit unabhängig von den verwendeten verdünnten basischen Fluiden und/oder verdünnten sauren Fluiden, insbesondere den verwendeten verdünnten sauren Fluiden.

Weiter kann der Parameter zur Umrechnung einer Natriumionenkonzentration in eine Leitfähigkeit der Dialysierflüssigkeit oder umgekehrt gemäß folgender Gleichung (4#) bestimmt werden: ${Λ}_{m , acid} = 0.09921 \frac{mS l}{mmol cm} \frac{{c}_{{Na}^{+} , 0} + {c}_{{K}^{+} , 0} + 2 ∗ {c}_{{Mg}^{+} , 0} + 2 ∗ {c}_{{Ca}^{+} , 0}}{{c}_{{Na}^{+} , 0}}$ (4#) wobei
- Λ*_{m,acid}*: dem Parameter zur Umrechnung einer Natriumionenkonzentration in eine Leitfähigkeit der Dialysierflüssigkeit oder umgekehrt,
- *c*_{*Na*⁺*,*0}: **einer** Natriumionenkonzentration des verdünnten sauren Fluids,
- *c*_{*K*⁺*,*0}: einer Kaliumionenkonzentration des verdünnten sauren Fluids,
- 2 * *c*_{*Mg*⁺*,*0}: einer Magnesiumionenkonzentration des verdünnten sauren Fluids und
- 2 * *c*_{*Ca*⁺,0}: einer Calciumionenkonzentration des verdünnten sauren Fluids entspricht.

In diesem Fall wird beim Durchführen von Schritt i) die Natriumionenkonzentration der Dialysierflüssigkeit mithilfe eines linearen Modells, insbesondere gemäß folgender Gleichung (9#) bestimmt: ${{c}^{+}}_{{Na}^{+} , gesamt} \left(mmol/l\right) = \left\{{ENDLF}^{+}-\left[{{c}^{+}}_{bic}\left(mmol/l\right)\times {Λ}_{m , bic}\right]\right\} / {Λ}_{m , acid , kor} + {{c}^{+}}_{bic} \left(mmol/l\right)$ (9#) wobei
- c⁺_{Na+,gesamt}: der Natriumionenkonzentration der Dialysierflüssigkeit,
- ENDLF⁺: der gemäß Schritt g) gemessenen Leitfähigkeit der Dialysierflüssigkeit,
- c⁺_{bic}: der Bicarbonationenkonzentration der Dialysierflüssigkeit,
- Λ*_{m,acid}*: dem Parameter zur Umrechnung einer Natriumionenkonzentration in eine Leitfähigkeit der Dialysierflüssigkeit oder umgekehrt und
- Λ*_{m,bic}*: dem Parameter zur Umrechnung einer Bicarbontationenkonzentration in eine Bicarbonatleitfähigkeit oder umgekehrt entspricht.

**Der von einem** Nutzer in eine Vorrichtung zur extrakorporalen Blutbehandlung, insbesondere Dialysemaschine, eingegebene Set-Wert für die Bicarbonationenkonzentration wird von der Vorrichtung zur extrakorporalen Blutbehandlung als Bicarbonationenkonzentration nach Neutralisierung mit der Säure des verdünnten sauren Fluids, d.h. als Bicarbonationenkonzentration der Dialysierflüssigkeit, gewertet *c_{BIC,post}.* Aus dieser Konzentration wird die Bicarbonationenkonzentration vor Neutralisierung mit der Säure des verdünnten sauren Fluids, d.h. die Bicarbonationenkonzentration der Vorstufe der Dialysierflüssigkeit, *c_{BIC,pre}* gemäß folgender Gleichung (1*) bestimmt: ${c}_{bic , pre} = \frac{{c}_{bic , post} + \frac{{c}_{Na , set}}{{c}_{{Na}^{+} , 0} - {c}_{bic , 0} - {c}_{acid , 0}} {c}_{acid , 0}}{1 + \frac{{c}_{Na , set}}{{c}_{{c}_{{Na}^{+} , 0}} - {c}_{bic , 0} - {c}_{acid , 0}}}$ (1*) wobei
- *c_{bic,pre}*: der Bicarbonationenkonzentration der Vorstufe der Dialysierflüssigkeit,
- *c_{bic,post}*: einer für die Dialysierflüssigkeit vorgegebenen Bicarbonationenkonzentration,
- *c_{Na,set}*: einer für die Dialysierflüssigkeit vorgegebenen Natriumionenkonzentration,
- *c*_{*bic,*0}: einer Bicarbonationenkonzentration des verdünnten basischen Fluids,
- *c*_{*Na*⁺*,*0}: einer Natriumionenkonzentration des verdünnten sauren Fluids und
- *c*_{*acid,*0}: einer Säurekonzentration des verdünnten sauren Fluids entspricht.

Der Wert *c_{BIC,pre}* wird dann in die Gleichung (3) als Setwert für die Bicarbonatkonzentration eingesetzt. Hieraus ergibt sich der Parameter zur Umrechnung einer Bicarbontationenkonzentration in eine Bicarbonatleitfähigkeit oder umgekehrt unabhängig vom verwendeten verdünnten basischen Fluid und/oder verdünnten sauren Fluid, insbesondere verdünnten sauren Fluid, gemäß obiger Gleichung (3#). Der Parameter zur Umrechnung einer Natriumionenkonzentration in eine Leitfähigkeit der Dialysierflüssigkeit oder umgekehrt ergibt sich gemäß obiger Gleichung (4#).

Dadurch kann vorteilhafterweise die tatsächliche Konzentration weiterer Kationenspezies der Dialysierflüssigkeit, insbesondere ausgewählt aus der Gruppe bestehend aus Kaliumionenkonzentration, Magnesiumionenkonzentration, Calciumionenkonzentration und Kombinationen von wenigstens zwei der vorgenannten Kationenspezieskonzentrationen, bei einer für die Dialysierflüssigkeit vorgegebenen Bicarbonat- und Natriumionenkonzentrationen gemäß nachfolgender Gleichung (10) bestimmt werden: ${c}_{X , act} = \frac{{c}_{Na , set} - {c}_{bic , pre}}{{c}_{{Na}^{+} , 0} - {c}_{bic , 0} - {c}_{acid , 0}} {c}_{X , 0}$ (10) wobei
- *c_{X,act}*: der tatsächlichen Konzentration einer weiteren Kationenspezies der Dialysierflüssigkeit, insbesondere ausgewählt aus der Gruppe bestehend aus Kaliumionenkonzentration, Magnesiumionenkonzentration, Calciumionenkonzentration und Kombinationen von wenigstens zwei der vorgenannten Kationenspezieskonzentrationen,
- *c_{Na,set}*: einer für die Dialysierflüssigkeit vorgegebenen Natriumionenkonzentration,
- *c_{bic,pre}*: der Bicarbonationenkonzentration der Vorstufe der Dialysierflüssigkeit,
- *c*_{*X,*0}: einer Konzentration einer weiteren Kationenspezies des verdünnten sauren Fluids, insbesondere ausgewählt aus der Gruppe bestehend aus Kaliumionenkonzentration, Magnesiumionenkonzentration, Calciumionenkonzentration und Kombinationen von wenigstens zwei der vorgenannten Kationenspezieskonzentrationen,
- *c*_{*Na*⁺,0}: einer Natriumionenkonzentration des verdünnten sauren Fluids,
- *c*_{*acid,*0}: einer Säurekonzentration des verdünnten sauren Fluids entspricht und
- *c*_{*bic,*0}: einer Bicarbonationenkonzentration des verdünnten basischen Fluids entspricht.

Weiter kann vorteilhafterweise der Gesamtpuffer (Total Buffer) der Dialysierflüssigkeit bei einer für die Dialysierflüssigkeit vorgegebenen Bicarbonat- und Natriumionenkonzentration bestimmt werden.

Der Gesamtpuffer kann dabei in Abhängigkeit des ausgewählten verdünnten sauren Fluids angegeben werden:

### Essigsäure und Zitronensäure (Beispiel SW 380, Citradial):

In diesem Fall ist der Gesamtpuffer identisch mit der Bicarbonationenkonzentration vor Neutralisation mit der Säure des verdünnten sauren Fluids, d.h. mit der Bicarbonationenkonzentration der Vorstufe der Dialysierflüssigkeit, da für jedes neutralisierte Bicarbonat ein Säuresalzpendant erstellt wird: $Gesamtpuffer = {c}_{BIC , pre}$ (11)

### Salzsäure (Beispiel Lympha)

In diesem Fall ist der Gesamtpuffer identisch mit der Bicarbonationenkonzentration nach Neutralisation mit der Säure des verdünnten sauren Fluids, d.h. mit der Bicarbonationenkonzentration der Dialysierflüssigkeit. Bei Salzsäure entsteht als Salz der Säure lediglich Chlorid, welches im Körper nicht wie Acetat und Citrat zu Bicarbonat verstoffwechselt wird. $Gesamtpuffer = {c}_{bic , post}$ (12)

### Acetessigsäure (Beispiel Granuflo)

In diesem Fall wird für jede zugefügte Essigsäure ein weiteres Acetat hinzugefügt, welches zu Bicarbonat verstoffwechselt werden kann. $Gesamtpuffer = {c}_{bic , pre} + \frac{{c}_{Na , set} - {c}_{bic , pre}}{{c}_{{Na}^{+} , 0} - {c}_{bic , 0} - {c}_{acid , 0}} {c}_{acid , 0}$ (13) wobei
- *c_{bic,pre}*: der Bicarbonationenkonzentration der Vorstufe der Dialysierflüssigkeit,
- *c_{Na,set}*: einer für die Dialysierflüssigkeit vorgegebenen Natriumionenkonzentration,
- *c*_{*Na*₊*,*0}: einer Natriumionenkonzentration des verdünnten sauren Fluids,
- *c*_{*acid,*0}: einer Säurekonzentration des verdünnten sauren Fluids und
- *c*_{*bic,*0}: einer Bicarbonationenkonzentration des verdünnten basischen Fluids entspricht.

Das Verfahren kann daher einen weiteren Schritt j) Bestimmen der Konzentration wenigstens eines weiteren Bestandteils der Dialysierflüssigkeit aufweisen, wobei der wenigstens eine weitere Bestandteil ausgewählt ist aus der Gruppe bestehend aus Kaliumionen, Magnesiumionen, Calciumionen, Gesamtpuffer und Kombinationen von wenigstens zwei der vorgenannten Bestandteile der Dialysierflüssigkeit.

In weiterer Ausgestaltung der Erfindung werden die Konzentrationen des basischen Fluids und/oder des sauren Fluids auf einer Vorrichtung zur extrakorporalen Blutbehandlung, insbesondere auf einer Vorrichtung zur Hämodialyse oder Peritoneladialyse, bevorzugt auf einer Dialysemaschine, insbesondere Hämodialysemaschine oder Peritonealdialysemaschine, oder einem anderen System gespeichert oder hinterlegt.

In weiterer Ausgestaltung der Erfindung wird das Verfahren wenigstens teilweise, insbesondere nur teilweise oder vollständig, von einer Vorrichtung zur extrakorporalen Blutbehandlung, insbesondere einer Vorrichtung zur Hämodialyse oder Peritoneladialyse, bevorzugt einer Dialysemaschine, insbesondere Hämodialysemaschine oder Peritonealdialysemaschine, durchgeführt.

Weiter kann es bevorzugt sein, dass das Verfahren nur teilweise oder vollständig automatisiert durchgeführt wird. Mit anderen Worten kann es insbesondere bevorzugt sein, wenn nur ein Teil der Schritte, d.h. nur manche Schritte, oder alle Schritte des Verfahrens automatisiert durchgeführt werden.

In weiterer Ausgestaltung der Erfindung erfolgen/erfolgt abhängig von der gemäß Schritt i) bestimmten Bicarbonationenkonzentration eine weitere, insbesondere kontinuierliche oder diskontinuierliche, Zugabe des verdünnten basischen Fluids zu der Vorstufe der Dialysierflüssigkeit und/oder abhängig von der gemäß Schritt i) bestimmten Natriumionenkonzentration eine weitere, insbesondere kontinuierliche oder diskontinuierliche, Zugabe des verdünnten sauren Fluids zu der Dialysierflüssigkeit.

Gemäß einem zweiten Aspekt betrifft die Erfindung die Verwendung des Verfahrens gemäß erstem Erfindungsaspekt zur Herstellung oder Proportionierung einer/der Dialysierflüssigkeit, insbesondere für die Hämodialyse und/oder Peritonealdialyse, bevorzugt Hämodialyse.

Bezüglich weiterer Merkmale und Vorteile der Verwendung, insbesondere in Bezug auf das Verfahren, wird vollständig auf die unter dem ersten Erfindungsaspekt gemachten Ausführungen Bezug genommen. Die dort in Bezug auf das Verfahren beschriebenen Merkmale und Vorteile gelten sinngemäß auch für die Verwendung gemäß zweitem Erfindungsaspekt.

Gemäß einem dritten Aspekt betrifft die Erfindung eine Vorrichtung zur extrakorporalen Blutbehandlung, insbesondere zur Hämo- und/oder Peritonealdialyse, bevorzugt zur Hämodialyse, insbesondere zur Durchführung eines Verfahrens gemäß erstem Erfindungsaspekt, oder die Verwendung einer Vorrichtung zur extrakorporalen Blutbehandlung, insbesondere zur Hämo- und/oder Peritonealdialyse, bevorzugt zur Hämodialyse, zur Durchführung eines Verfahrens gemäß erstem Erfindungsaspekt.

Die Vorrichtung kann insbesondere eine erste Mischkammer zum Mischen von Wasser, d.h. hochreinem Wasser oder Osmosewasser, und einem verdünnten basischen Fluid unter Ausbildung einer Vorstufe einer Dialysierflüssigkeit und eine zweite Mischkammer zum Mischen von der Vorstufe der Dialysierflüssigkeit und einem verdünnten sauren Fluid unter Ausbildung einer Dialysierflüssigkeit aufweisen.

Weiter kann die Vorrichtung eine erste Förderpumpe zum Fördern des verdünnten basischen Fluids in die erste Mischkammer und eine zweite Förderpumpe zum Fördern des verdünnten sauren Fluids in die zweite Mischkammer aufweisen.

Weiter kann die Vorrichtung einen Temperatursensor zum Messen einer Temperatur der Vorstufe der Dialysierflüssigkeit sowie einen Leitfähigkeitssensor zum Messen einer Leitfähigkeit der Vorstufe der Dialysierflüssigkeit aufweisen.

Weiter kann die Vorrichtung einen Temperatursensor zum Messen einer Temperatur der Dialysierflüssigkeit sowie einen Leitfähigkeitssensor zum Messen einer Leitfähigkeit der Dialysierflüssigkeit aufweisen.

Weiter kann die Vorrichtung eine Software zum Abgleichen einer gemessenen Leitfähigkeit der Vorstufe der Dialysierflüssigkeit mit einem Sollwert für eine Bicarbonationenleitfähigkeit der Dialysierflüssigkeit und/oder Vorstufe der Dialysierflüssigkeit sowie zum Abgleichen einer gemessenen Leitfähigkeit der Dialysierflüssigkeit mit einem Sollwert für eine Leitfähigkeit der Dialysierflüssigkeit aufweisen.

Weiter kann die Vorrichtung eine Steuereinrichtung zum Steuern einer Fördergeschwindigkeit des verdünnten basischen Fluids in die erste Mischkammer in Abhängigkeit einer gemessenen Leitfähigkeit der Vorstufe der Dialysierflüssigkeit sowie zum Steuern einer Fördergeschwindigkeit des verdünnten sauren Fluids in die zweite Mischkammer in Abhängigkeit einer gemessenen Leitfähigkeit der Dialysierflüssigkeit aufweisen.

Die vorgenannten Komponenten der Vorrichtung können insbesondere Bestandteil einer Dialysierflüssigkeitsbereitstellungseinheit der Vorrichtung sein.

Bevorzugt ist die Vorrichtung zur extrakorporalen Blutbehandlung als Dialysemaschine, insbesondere für die Hämodialyse und/oder Peritonealdialyse, bevorzugt Hämodialyse, gestaltet.

Bezüglich weiterer Merkmale und Vorteile der Vorrichtung oder Verwendung der Vorrichtung, insbesondere in Bezug auf das Verfahren, wird vollständig auf die unter dem ersten Erfindungsaspekt gemachten Ausführungen Bezug genommen. Die dort in Bezug auf das Verfahren beschriebenen Merkmale und Vorteile gelten sinngemäß auch für die Vorrichtung oder Verwendung der Vorrichtung gemäß drittem Erfindungsaspekt.

An dieser Stelle sollen die Vorteile des erfindungsgemäßen Verfahrens noch einmal wie folgt zusammengefasst werden:
Mit dem erfindungsgemäßen Verfahren lassen sich Umrechnungsfaktoren für basische Fluide (basische Konzentrate bzw. Bicarbonatkonzentrate) und saure Fluide (Säurekonzentrate) ohne experimentelle Laborbestimmung lediglich auf Basis der Standardkonzentration der basischen Fluide und/oder sauren Fluide, insbesondere nur der sauren Fluide, bestimmen. Die Faktoren sind optimiert auf die Standardkonzentrationen der basischen Fluide und/oder sauren Fluide, insbesondere nur der sauren Fluide. Der experimentell bestimmte Modellfehler liegt in diesem Bereich vorteilhafterweise kleiner 1 mmol/I für Natrium- und Bicarbonationen.

In einer Vorrichtung zur extrakorporalen Blutbehandlung, insbesondere einer Dialysemaschine, müssen somit keine Umrechnungsfaktoren mehr hinterlegt werden, welche zuvor ein Techniker bestimmen musste. Vielmehr müssen lediglich die Standardkonzentrationen der verwendeten basischen Fluide und/oder sauren Fluide, insbesondere der sauren Fluide, im Gerät konfiguriert werden. Eine solche Aktivität erfordert keinen ausgebildeten Techniker und kann beispielsweise von einer Dialyseschwester durchgeführt werden.

Weitere Merkmale und Vorteile der Erfindung ergeben sich aus den Ansprüchen sowie aus der nachfolgenden Beschreibung bevorzugter Ausführungsbeispiele der Erfindung, die anhand der Zeichnungen schematisch dargestellt sind. Es versteht sich, dass die Erfindung hierauf nicht beschränkt sein soll.
- Fig. 1: zeigt eine Dialysierflüssigkeitsbereitstellungseinheit einer Dialysemaschine zur Durchführung des erfindungsgemäßen Verfahrens,
- Fig. 2: einen Closed-Loop-Controller zur Einstellung einer Fördergeschwindigkeit einer Pumpe zum Fördern des verdünnten basischen Fluids,
- Fig. 3: einen Closed-Loop-Controller zum Einstellen einer Fördergeschwindigkeit einer Pumpe zum Fördern des verdünnten sauren Fluids,
- Fig. 4: die Bestimmung der Sollwerte für die Bicarbonatleitfähigkeit der Vorstufe der Dialysierflüssigkeit sowie der Leitfähigkeit der Dialysierflüssigkeit,
- Fig. 5: ein User Interface einer Dialysemaschine zur Durchführung des erfindungsgemäßen Verfahrens und
- Fig. 6: ein Ablaufschema eines erfindungsgemäßen Verfahrens.

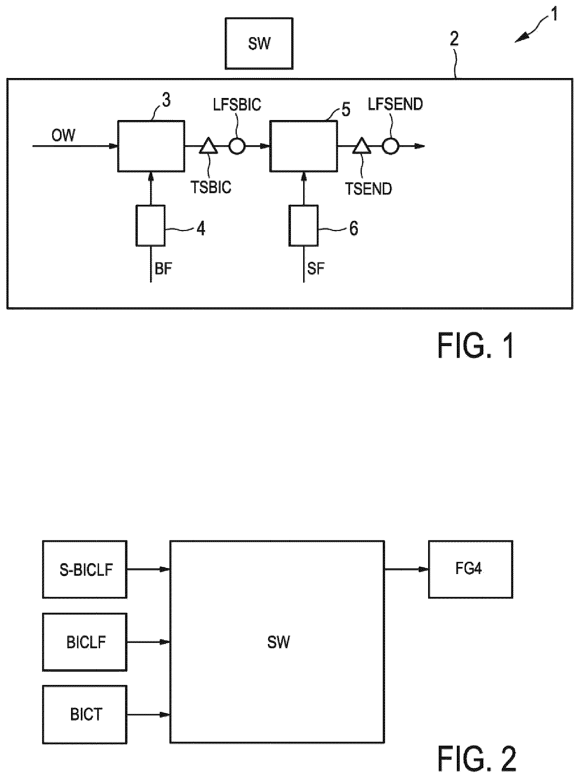

Fig. 1 zeigt schematisch eine Ausführungsform einer Vorrichtung 1 zur Durchführung des erfindungsgemäßen Verfahrens.

Die Vorrichtung 1 weist eine Dialysierflüssigkeitsbereitstellungseinheit 2 auf.

In der Dialysierflüssigkeitsbereitstellungseinheit 2 werden Osmosewasser, ein durch Mischen eines basischen Fluids, das eine definierte oder nicht definierte Bicarbonationenkonzentration aufweist, mit Wasser verdünntes basisches Fluid sowie ein durch Mischen eines sauren Fluids, das eine definierte oder nicht definierte Natriumionenkonzentration sowie eine definierte oder nicht definierte Säurekonzentration aufweist, mit Wasser verdünntes saures Fluid zu einer Dialysierflüssigkeit vermischt. Die Dialysierflüssigkeit wird, gegebenenfalls zu einem späteren Zeitpunkt, in einen (nicht dargestellten) Dialysator der Vorrichtung 1 gefördert.

### Das Bereitstellen oder Herstellen der Dialysierflüssigkeit erfolgt dabei Folgendermaßen:

Zunächst werden das Osmosewasser OW und das verdünnte basische Fluid BF in einer ersten Mischkammer 3 der Dialysierflüssigkeitsbereitstellungseinheit 2 gemischt. Das verdünnte basische Fluid BF wird hierzu über eine, insbesondere volumetrisch gesteuerte, Förderpumpe 4 in die erste Mischkammer 3 gefördert. In der ersten Mischkammer 3 findet eine ideale Mischung zwischen dem Osmosewasser OW und dem verdünnten basischen Fluid BF zu einer Vorstufe der Dialysierflüssigkeit statt. Die Vorstufe der Dialysierflüssigkeit passiert sodann einen Temperatursensor TSBIC und einen Leitfähigkeitssensor LFSBIC. Der Temperatursensor TSBIC ist dazu ausgebildet, eine Temperatur BICT der Vorstufe der Dialysierflüssigkeit zu messen. Der Leitfähigkeitssensor LFSBIC ist dazu ausgebildet, eine Leitfähigkeit BICLF der Vorstufe der Dialysierflüssigkeit zu messen. Vorzugsweise werden die mittels der vorgenannten Sensoren bestimmten Messwerte BICT und BICLF von einer Software SW ausgewertet, um eine temperaturkompensierte Leitfähigkeit der Vorstufe der Dialysierflüssigkeit zu bestimmen.

Im weiteren Verlauf des erfindungsgemäßen Verfahrens wird die Leitfähigkeit mit einem Sollwert für die Bicarbonatleitfähigkeit S-BICLF der Dialysierflüssigkeit und/oder Vorstufe der Dialysierflüssigkeit mittels der Software SW verglichen. Je nach gemessener Leitfähigkeit BICLF erfolgt ein kontinuierliches Anpassen der Fördergeschwindigkeit FG4 der Förderpumpe 4 über die Software SW, um die Leitfähigkeit BICLF der Vorstufe der Dialysierflüssigkeit möglichst nahe an den Sollwert für die Bicarbonatleitfähigkeit S-BICLF anzunähern (siehe den in Fig. 2 schematisch dargestellten Closed-Loop-Controller).

In einem nächsten Schritt wird der Vorstufe der Dialysierflüssigkeit das verdünnte saure Fluid SF in einer zweiten Mischkammer 5 der Dialysierflüssigkeitsbereitstellungseinheit 2 beigemischt. Hierzu wird das verdünnte saure Fluid SF über eine, insbesondere volumetrisch gesteuerte, Förderpumpe 6 in die zweite Mischkammer 5 gefördert. In der zweiten Mischkammer 5 findet eine ideale Mischung zwischen der Vorstufe der Dialysierflüssigkeit und dem verdünnten sauren Fluid SF zu der Dialysierflüssigkeit statt. Die Dialysierflüssigkeit passiert sodann einen Temperatursensor TSEND und einen Leitfähigkeitssensor LFSEND. Der Temperatursensor TSEND ist dazu ausgebildet, eine Temperatur ENDT der Dialysierflüssigkeit zu messen. Der Leitfähigkeitssensor LFSEND ist dazu ausgebildet, eine Leitfähigkeit ENDLF der Dialysierflüssigkeit zu messen. Vorzugsweise werden die über die beiden vorgenannten Sensoren bestimmten Messwerte ENDT und ENDLF von der Software SW ausgewertet, um eine temperaturkompensierte Leitfähigkeit ENDLF zu bestimmen.

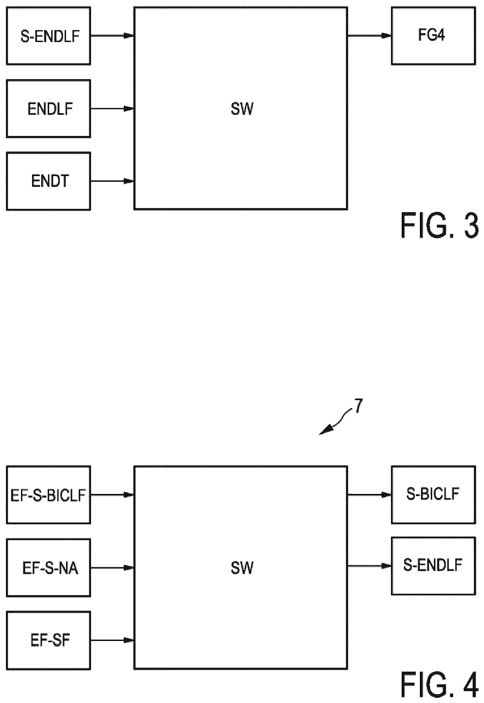

Im weiteren Verlauf des erfindungsgemäßen Verfahrens wird die Leitfähigkeit ENDLF der Dialysierflüssigkeit mittels der Software SW mit einem Sollwert für die Leitfähigkeit S-ENDLF der Dialysierflüssigkeit verglichen. Je nach gemessener Leitfähigkeit ENDLF erfolgt eine kontinuierliche Anpassung der Fördergeschwindigkeit FG6 der Förderpumpe 6 über die Software SW, um die Leitfähigkeit ENDLF möglichst nahe an den Sollwert für die Leitfähigkeit S-ENDLF der Dialysierflüssigkeit anzunähern (siehe den in Fig. 3 dargestellten Closed-Loop-Controller).

Bevorzugt können Software und Sensorik der Vorrichtung 1 aus Sicherheitsgründen zweikanalig redundant ausgelegt sein (ein zweiter Kanal ist aus Gründen der Übersichtlichkeit in den Fig. 2 und 3 nicht dargestellt).

Die für die beiden Closed-Loop-Controller benötigten Sollwerte für die Bicarbonatleitfähigkeit und die Leitfähigkeit der Dialysierflüssigkeit werden von der Software SW mittels der in der allgemeinen Beschreibung erwähnten Gleichungen (1) und (2) bestimmt. Hierzu werden als Input Sollkonzentrationen für Natriumionen und Bicarbonationen sowie die gewünschten basischen Fluide und sauren Fluide verwendet, welche von einem Nutzer der Vorrichtung 1 spezifisch zur Durchführung einer extrakorporalen Blutbehandlung in die Vorrichtung 1 eingegeben werden können.

Hierzu kann die Vorrichtung 1 ein entsprechendes User Interface aufweisen. Ein geeignetes User Interface 7 ist schematisch in Fig. 4 gezeigt. Das User Interface 7 weist ein Eingabefeld EF-S-BICLF für einen Sollwert der Bicarbonatkonzentration der Dialysierflüssigkeit und/oder der Vorstufe der Dialysierflüssigkeit, ein Eingabefeld EF-S-NA für einen Sollwert der Natriumionenkonzentration der Dialysierflüssigkeit sowie ein Eingabefeld EF-SF für das verdünnte saure Fluid auf. Aus den vorgenannten Sollwerten ermittelt die Software SW sodann einen Sollwert für die Bicarbonatleitfähigkeit S-BICLF der Vorstufe der Dialysierflüssigkeit sowie einen Sollwert für die Leitfähigkeit S-ENDLF der Dialysierflüssigkeit.

Etwaige weitere Parameter, welche zur Berechnung des Sollwerts für die Bicarbonatleitfähigkeit S-BICLF und des Sollwerts für die Leitfähigkeit S-ENDLF der Dialysierflüssigkeit benötigt werden, können vorteilhafterweise in der Vorrichtung 1 hinterlegt sein, so dass sie nicht bei jeder Behandlung eingegeben werden müssen. Beispielsweise könnten entsprechende weitere Parameter ex factory in der Software SW verfügbar sein oder vor Ort im Vorfeld einer Behandlung konfiguriert werden.

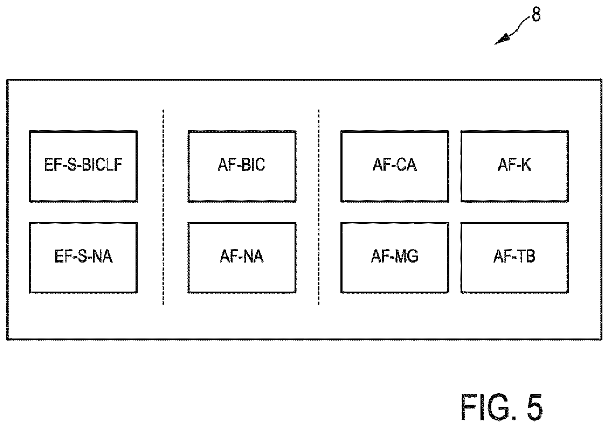

Aus den gemessenen, insbesondere temperaturkompensierten, Leitfähigkeiten können die aktuellen Konzentrationen der Dialysierflüssigkeitsbestandteile errechnet werden. Diese können wiederum neben Einstellparametern auf einem User Interface der Vorrichtung 1 angezeigt werden. Ein entsprechendes User Interface st schematisch in der Fig. 5 dargestellt. Das in Fig. 5 dargestellte User Interface 8 weist ein Eingabefeld EF-S-BICLF für einen Sollwert der Bicarbonationenkonzentration der Dialysierflüssigkeit und/oder Vorstufe der Dialysierflüssigkeit, ein Eingabefeld EF-S-NA für einen Sollwert der Natriumionenkonzentration der Dialysierflüssigkeit, ein Anzeigefeld AF-BIC für eine aktuelle Bicarbonatkonzentration der Dialysierflüssigkeit und/oder Vorstufe der Dialysierflüssigkeit und ein Anzeigefeld AF-NA für eine aktuelle Natriumionenkonzentration der Dialysierflüssigkeit auf. Optional kann das User Interface zudem ein Anzeigefeld AF-CA für eine aktuelle Calciumionenkonzentration der Dialysierflüssigkeit, ein Anzeigefeld AF-K einer aktuellen Kaliumionenkonzentration der Dialysierflüssigkeit, ein Anzeigefeld AF-MG für eine aktuelle Magnesiumionenkonzentration der Dialysierflüssigkeit sowie ein Anzeigefeld AF-TB einer aktuellen Gesamtpufferkonzentration der Dialysierflüssigkeit aufweisen.

Hierzu können die in der allgemeinen Beschreibung genannten Gleichungen (1), (2), (10), (11), (12) und (13) verwendet werden.

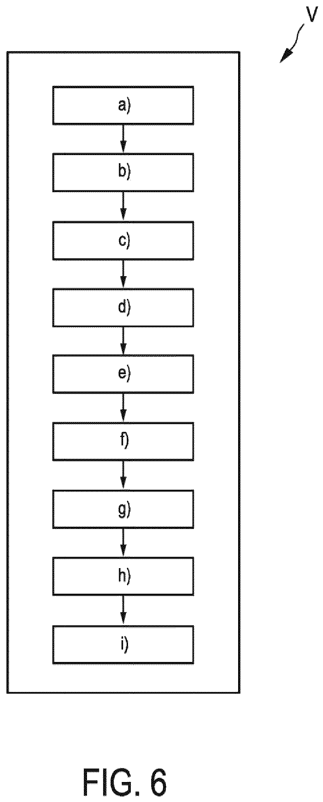

Fig. 6 zeigt ein Ablaufschema oder Ablaufdiagramm des erfindungsgemäßen Verfahrens V.

Bei dem erfindungsgemäßen Verfahren V handelt sich um ein Verfahren zum Bestimmen einer Natriumionenkonzentration und Bicarbonationenkonzentration einer Dialysierflüssigkeit, insbesondere für die Hämodialyse und/oder Peritonealdialyse.

Das Verfahren weist die nachfolgenden Schritte auf. Dabei können die nachfolgenden Schritte in zeitlicher Reihenfolge oder, insbesondere wenigstens teilweise, nicht in zeitlicher Reihenfolge durchgeführt werden.

Gemäß einem Schritt a) ein verdünntes basisches Fluid durch Mischen eines basischen Fluids, das eine, insbesondere definierte oder nicht definierte, Bicarbonationenkonzentration (Hydrogencarbonationenkonzentration) aufweist, mit Wasser, d.h. hochreinem Wasser oder Osmosewasser, gemäß einem definierten Mischungsverhältnis sowie ein verdünntes saures Fluid durch Mischen eines sauren Fluids, das eine, insbesondere definierte oder nicht definierte, Natriumionenkonzentration sowie eine, insbesondere definierte oder nicht definierte, Säurekonzentration aufweist, mit Wasser, d.h. hochreinem Wasser oder Osmosewasser, gemäß einem definierten Mischungsverhältnis bereitgestellt.

Neben der Bicarbonationenkonzentration kann das basische Fluid ferner eine, insbesondere definierte oder nicht definierte, Natriumionenkonzentration aufweisen. Insbesondere kann das basische Fluid in Form einer wässrigen Natriumhydrogencarbonatlösung bereitgestellt werden.

Das saure Fluid kann neben der Natriumionenkonzentration ferner eine, insbesondere definierte oder nicht definierte, Kaliumionenkonzentration, eine, insbesondere definierte oder nicht definierte, Magnesiumionenkonzentration und eine, insbesondere definierte oder nicht definierte, Calciumionenkonzentration aufweisen.

Insbesondere kann das saure Fluid in Form einer wässrigen sauren Lösung bereitgestellt werden, die Natriumchlorid, Kaliumchlorid, Magnesiumchlorid, Calciumchlorid und eine Säure, insbesondere Essigsäure oder Zitronensäure, aufweist. Ggf. kann das saure Fluid zusätzlich eine osmotisch wirksame Verbindung, insbesondere Glucose, aufweisen.

Gemäß einem Schritt b) wird eine Vorstufe der Dialysierflüssigkeit durch, insbesondere kontinuierliche oder diskontinuierliche, Zugabe des verdünnten basischen Fluids zu Wasser, d.h. hochreinem Wasser oder Osmosewasser, hergestellt.

Gemäß einem Schritt c) werden ein Sollwert für eine Bicarbonatleitfähigkeit der Dialysierflüssigkeit und/oder Vorstufe der Dialysierflüssigkeit auf Basis einer, insbesondere definierten oder nicht definierten, Bicarbonationenkonzentration für die Dialysierflüssigkeit und/oder Vorstufe der Dialysierflüssigkeit und eines Parameters zur Umrechnung einer Bicarbonationenkonzentration in eine Bicarbonatleitfähigkeit oder umgekehrt, d.h. zur Umrechnung einer Bicarbonatleitfähigkeit in eine Bicarbonationenkonzentration, und ein Sollwert für eine Leitfähigkeit, insbesondere End- oder Gesamtleitfähigkeit, der Dialysierflüssigkeit auf Basis einer, insbesondere definierten oder nicht definierten, Natriumionenkonzentration für die Dialysierflüssigkeit und eines Parameters zur Umrechnung einer Natriumionenkonzentration in eine Leitfähigkeit, insbesondere End- oder Gesamtleitfähigkeit, der Dialysierflüssigkeit oder umgekehrt, d.h. zur Umrechnung einer Leitfähigkeit, insbesondere End- oder Gesamtleitfähigkeit, der Dialysierflüssigkeit in eine Natriumionenkonzentration bestimmt.

Gemäß einem Schritt d) wird eine Leitfähigkeit der Vorstufe der Dialysierflüssigkeit gemessen.

Gemäß einem Schritt e) wird die gemäß Schritt d) gemessene Leitfähigkeit der Vorstufe der Dialysierflüssigkeit mit dem gemäß Schritt c) bestimmten Sollwert für die Bicarbonatleitfähigkeit der Dialysierflüssigkeit und/oder Vorstufe der Dialysierflüssigkeit abgeglichen und gegebenenfalls wird von dem verdünnten basischen Fluid, insbesondere kontinuierlich oder diskontinuierlich, zusätzlich zu der Vorstufe der Dialysierflüssigkeit, insbesondere mittels einer Förderpumpe, zugegeben, bis der Sollwert für die Bicarbonatleitfähigleit der Dialysierflüssigkeit und/oder Vorstufe der Dialysierflüssigkeit erreicht oder im Wesentlichen erreicht ist.

Gemäß einem Schritt f) wird die Dialysierflüssigkeit durch, insbesondere kontinuierliche oder diskontinuierliche, Zugabe des verdünnten sauren Fluids zu der Vorstufe der Dialysierflüssigkeit hergestellt.

Gemäß einem Schritt g) wird eine Leitfähigkeit, insbesondere End- oder Gesamtleitfähigkeit, der Dialysierflüssigkeit gemessen.

Gemäß einem Schritt h) wird die gemäß Schritt g) gemessene Leitfähigkeit der Dialysierflüssigkeit mit dem gemäß Schritt c) bestimmten Sollwert für die Leitfähigkeit der Dialysierflüssigkeit abgeglichen und gegebenenfalls wird von dem verdünnten sauren Fluid, insbesondere kontinuierlich oder diskontinuierlich, zusätzlich zu der Dialysierflüssigkeit zugegeben, insbesondere mittels einer weiteren Förderpumpe, bis der Sollwert für die Leitfähigkeit der Dialysierflüssigkeit erreicht oder im Wesentlichen erreicht ist.

Gemäß einem Schritt i) werden die Bicarbonationenkonzentration der Dialysierflüssigkeit basierend auf der gemäß Schritt d) gemessenen Leitfähigkeit der Vorstufe der Dialysierflüssigkeit und des Parameters zur Umrechnung einer Bicarbonationenkonzentration in eine Bicarbonatleitfähigkeit oder umgekehrt, d.h. zur Umrechnung einer Bicarbonatleitfähigkeit in eine Bicarbonationenkonzentration, sowie die Natriumionenkonzentration der Dialysierflüssigkeit basierend auf der gemäß Schritt g) gemessenen Leitfähigkeit der Dialysierflüssigkeit und des Parameters zur Umrechnung einer Natriumionenkonzentration in eine Leitfähigkeit der Dialysierflüssigkeit oder umgekehrt, d.h. zur Umrechnung einer Leitfähigkeit der Dialysierflüssigkeit in eine Natriumionenkonzentration, bestimmt.

Das Verfahren V zeichnet sich insbesondere dadurch aus, dass der Parameter zur Umrechnung einer Bicarbonationenkonzentration in eine Bicarbonatleitfähigkeit oder umgekehrt, d.h. zur Umrechnung einer Bicarbonatleitfähigkeit in eine Bicarbonationenkonzentration, und der Parameter zur Umrechnung einer Natriumionenkonzentration in eine Leitfähigkeit der Dialysierflüssigkeit oder umgekehrt, d.h. zur Umrechnung einer Leitfähigkeit der Dialysierflüssigkeit in eine Natriumionenkonzentration, jeweils automatisiert ermittelt werden.

Bezüglich weiterer Merkmale und Vorteile des Verfahrens V wird vollständig auf die in der allgemeinen Beschreibung gemachten Ausführungen Bezug genommen.

## Patentansprüche

1. Verfahren zum Bestimmen einer Natrium- und Bicarbonationenkonzentration einer Dialysierflüssigkeit, wobei das Verfahren folgende Schritte aufweist:
a) Bereitstellen eines verdünnten basischen Fluids durch Mischen eines basischen Fluids, das eine Bicarbonationenkonzentration aufweist, mit Wasser gemäß einem definierten Mischungsverhältnis und Bereitstellen eines verdünnten sauren Fluids durch Mischen eines sauren Fluids, das eine Natriumionenkonzentration sowie eine Säurekonzentration aufweist, mit Wasser gemäß einem definierten Mischungsverhältnis,
b) Herstellen einer Vorstufe der Dialysierflüssigkeit durch Zugabe des verdünnten basischen Fluids zu Wasser,
c) Bestimmen eines Sollwerts für eine Bicarbonatleitfähigkeit der Dialysierflüssigkeit und/oder Vorstufe der Dialysierflüssigkeit auf Basis einer Bicarbonationenkonzentration für die Dialysierflüssigkeit und/oder Vorstufe der Dialysierflüssigkeit und eines Parameters zur Umrechnung einer Bicarbonationenkonzentration in eine Bicarbonatleitfähigkeit oder umgekehrt und Bestimmen eines Sollwerts für eine Leitfähigkeit der Dialysierflüssigkeit auf Basis einer Natriumionenkonzentration für die Dialysierflüssigkeit und eines Parameters zur Umrechnung einer Natriumionenkonzentration in eine Leitfähigkeit der Dialysierflüssigkeit oder umgekehrt,
d) Messen einer Leitfähigkeit der Vorstufe der Dialysierflüssigkeit,
e) Abgleichen der gemäß Schritt d) gemessenen Leitfähigkeit der Vorstufe der Dialysierflüssigkeit mit dem gemäß Schritt c) bestimmten Sollwert für die Bicarbonatleitfähigkeit der Dialysierflüssigkeit und/oder Vorstufe der Dialysierflüssigkeit und gegebenenfalls weitere Zugabe des verdünnten basischen Fluids zu der Vorstufe der Dialysierflüssigkeit, bis der Sollwert für die Bicarbonatleitfähigleit der Dialysierflüssigkeit und/oder Vorstufe der Dialysierflüssigkeit erreicht oder im Wesentlichen erreicht ist,
f) Herstellen der Dialysierflüssigkeit durch Zugabe des verdünnten sauren Fluids zu der Vorstufe der Dialysierflüssigkeit,
g) Messen einer Leitfähigkeit der Dialysierflüssigkeit,
h) Abgleichen der gemäß Schritt g) gemessenen Leitfähigkeit der Dialysierflüssigkeit mit dem gemäß Schritt c) bestimmten Sollwert für die Leitfähigkeit der Dialysierflüssigkeit und gegebenenfalls weitere Zugabe des verdünnten sauren Fluids zu der Dialysierflüssigkeit, bis der Sollwert für die Leitfähigkeit der Dialysierflüssigkeit erreicht oder im Wesentlichen erreicht ist, und
i) Bestimmen der Bicarbonationenkonzentration der Dialysierflüssigkeit basierend auf der gemäß Schritt d) gemessenen Leitfähigkeit der Vorstufe der Dialysierflüssigkeit und des Parameters zur Umrechnung einer Bicarbonationenkonzentration in eine Bicarbonatleitfähigkeit oder umgekehrt sowie Bestimmen der Natriumionenkonzentration der Dialysierflüssigkeit basierend auf der gemäß Schritt g) gemessenen Leitfähigkeit der Dialysierflüssigkeit und des Parameters zur Umrechnung einer Natriumionenkonzentration in eine Leitfähigkeit der Dialysierflüssigkeit oder umgekehrt,
wobei der Parameter zur Umrechnung einer Bicarbonationenkonzentration in eine Bicarbonatleitfähigkeit oder umgekehrt und der Parameter zur Umrechnung einer Natriumionenkonzentration in eine Leitfähigkeit der Dialysierflüssigkeit oder umgekehrt jeweils automatisiert ermittelt werden.

2. Verfahren nach Anspruch 1, **dadurch gekennzeichnet, dass** das basische Fluid ferner eine Natriumionenkonzentration aufweist, insbesondere in Form einer wässrigen basischen Lösung bereitgestellt wird, die Natriumhydrogencarbonat aufweist.

3. Verfahren nach Anspruch 1 oder 2, **dadurch gekennzeichnet, dass** das saure Fluid ferner eine Kaliumionenkonzentration, eine Magnesiumionenkonzentration und eine Calciumionenkonzentration aufweist, insbesondere in Form einer wässrigen sauren Lösung bereitgestellt wird, die Natriumchlorid, Kaliumchlorid, Magnesiumchlorid, Calciumchlorid und eine Säure, insbesondere Essigsäure, aufweist.

4. Verfahren nach einem der vorhergehenden Ansprüche, **dadurch gekennzeichnet, dass** der Sollwert für die Bicarbonatleitfähigkeit der Dialysierflüssigkeit und/oder Vorstufe der Dialysierflüssigkeit mithilfe eines linearen Modells, insbesondere gemäß folgender Gleichung (1) bestimmt wird: $BICLF = {c}_{bic} \left(mmol/l\right) \times {Λ}_{m , bic}$ (1) wobei
BICLF dem Sollwert für die Bicarbonatleitfähigkeit der Dialysierflüssigkeit und/oder Vorstufe der Dialysierflüssigkeit,
c_{bic} der Bicarbonationenkonzentration für die Dialysierflüssigkeit und/oder Vorstufe der Dialysierflüssigkeit und
Λ*_{m,bic}* dem Parameter zur Umrechnung einer Bicarbonationenkonzentration in eine Bicarbonatleitfähigkeit oder umgekehrt entspricht.

5. Verfahren nach einem der vorhergehenden Ansprüche, **dadurch gekennzeichnet, dass** der Sollwert für die Leitfähigkeit der Dialysierflüssigkeit mithilfe eines linearen Modells, insbesondere gemäß folgender Gleichung (2) bestimmt wird: $ENDLF = \left\{\left[{c}_{Na + , gesamt}\left(mmol\left(l\right)-{c}_{bic}\left(mmol/l\right)\right.\right]\times {Λ}_{m , acid}\right\} + \left[{c}_{bic}\left(mmol/l\right)\times {Λ}_{m , bic}\right]$ (2) wobei
ENDLF dem Sollwert für die Leitfähigkeit der Dialysierflüssigkeit,
c_{Na+,gesamt} der Natriumionenkonzentration für die Dialysierflüssigkeit,
c_{bic} der Bicarbonationenkonzentration für die Dialysierflüssigkeit und/oder Vorstufe der Dialysierflüssigkeit,
Λ_{*m*,*acid*} dem Parameter zur Umrechnung einer Natriumionenkonzentration in eine Leitfähigkeit der Dialysierflüssigkeit oder umgekehrt und
Λ*_{m,bic}* dem Parameter zur Umrechnung einer Bicarbonationenkonzentration in eine Bicarbonatleitfähigkeit oder umgekehrt entspricht.

6. Verfahren nach einem der vorhergehenden Ansprüche, **dadurch gekennzeichnet, dass** der Parameter zur Umrechnung einer Bicarbonationenkonzentration in eine Bicarbonatleitfähigkeit oder umgekehrt sowie der Parameter zur Umrechnung einer Natriumionenkonzentration in eine Leitfähigkeit der Dialysierflüssigkeit oder umgekehrt auf Basis von Konzentrationen des verdünnten basischen Fluids und/oder des verdünnten sauren Fluids, bevorzugt des verdünnten sauren Fluids, automatisiert ermittelt werden.

7. Verfahren nach einem der vorhergehenden Ansprüche, **dadurch gekennzeichnet, dass** der Parameter zur Umrechnung einer Bicarbonationenkonzentration in eine Bicarbonatleitfähigkeit oder umgekehrt auf Basis einer Bicarbonationenkonzentration des verdünnten basischen Fluids und einer Säurekonzentration des verdünnten sauren Fluids automatisiert ermittelt wird.

8. Verfahren nach einem der vorhergehenden Ansprüche, **dadurch gekennzeichnet, dass** der Parameter zur Umrechnung einer Natriumionenkonzentration in eine Leitfähigkeit der Dialysierflüssigkeit oder umgekehrt auf Basis einer Bicarbonationenkonzentration des verdünnten basischen Fluids, einer Natriumionenkonzentration des verdünnten basischen Fluids, einer Natriumionenkonzentration des verdünnten sauren Fluids, einer Kaliumionenkonzentration des verdünnten sauren Fluids, einer Magnesiumionenkonzentration des verdünnten sauren Fluids, einer Calciumionenkonzentration des verdünnten sauren Fluids und einer Säurekonzentration des verdünnten sauren Fluids automatisiert ermittelt wird.

9. Verfahren nach einem der vorhergehenden Ansprüche, **dadurch gekennzeichnet, dass** der Parameter zur Umrechnung einer Bicarbonationenkonzentration in eine Bicarbonatleitfähigkeit oder umgekehrt gemäß folgender Gleichung (3) bestimmt wird: ${Λ}_{m , bic} = 0.0852 \frac{mSl}{mmol cm} \frac{{c}_{bic , 0} + {c}_{acid , 0} \left[\frac{mEq}{l}\right]}{{c}_{bic , 0}}$ (3) wobei
Λ*_{m,bic}* dem Parameter zur Umrechnung einer Bicarbonationenkonzentration in eine Bicarbonatleitfähigkeit oder umgekehrt,
*c*_{*bic,*0} einer Bicarbonationenkonzentration des verdünnten basischen Fluids und
*c*_{*acid*,0} einer Säurekonzentration des verdünnten sauren Fluids entspricht.

10. Verfahren nach einem der vorhergehenden Ansprüche, **dadurch gekennzeichnet, dass** der Parameter zur Umrechnung einer Natriumionenkonzentration in eine Leitfähigkeit der Dialysierflüssigkeit oder umgekehrt gemäß folgender Gleichung (4) bestimmt wird: ${Λ}_{m , acid , kor} = \frac{11 \frac{mS}{cm}}{{c}_{Na , {acid}^{+}} \left(11\frac{mS}{cm}\right) + {c}_{acid} \left(11\frac{mS}{cm}\right)}$ (4) wobei
Λ*_{m,acid,kor}* dem Parameter zur Umrechnung einer Natriumionenkonzentration in eine Leitfähigkeit der Dialysierflüssigkeit oder umgekehrt,
${c}_{Na , {acid}^{+}} \left(11\frac{mS}{cm}\right)$ einer Natriumionenkonzentration des verdünnten sauren Fluids entspricht, die eine Leitfähigkeit des verdünnten sauren Fluids von 11 mS/cm bewirkt, und
${c}_{acid} \left(11\frac{mS}{cm}\right)$ einer Säurekonzentration des verdünnten sauren Fluids entspricht, dessen Natriumionenkonzentration eine Leitfähigkeit des verdünnten sauren Fluids von 11 mS/cm bewirkt.

11. Verfahren nach Anspruch 10, **dadurch gekennzeichnet, dass** die Natriumionenkonzentration des verdünnten sauren Fluids, die eine Leitfähigkeit des verdünnten sauren Fluids von 11 mS/cm bewirkt, gemäß folgender Gleichung (5) bestimmt wird: ${c}_{Na , {acid}^{+}} \left(11\frac{mS}{cm}\right) = \frac{11 \frac{mS}{cm}}{{Λ}_{m , acid}}$ (5) wobei
${c}_{Na , {acid}^{+}} \left(11\frac{mS}{cm}\right)$ der Natriumionenkonzentration des verdünnten sauren Fluids entspricht, die eine Leitfähigkeit des verdünnten sauren Fluids von 11 mS/cm bewirkt, und
Λ*_{m,acid}* einem unkorrigierten Parameter zur Umrechnung einer Natriumionenkonzentration in eine Leitfähigkeit der Dialysierflüssigkeit oder umgekehrt entspricht.

12. Verfahren nach Anspruch 11, **dadurch gekennzeichnet, dass** der unkorrigierte Parameter Λ*_{m,acid}* zur Umrechnung einer Natriumionenkonzentration in eine Leitfähigkeit der Dialysierflüssigkeit oder umgekehrt gemäß folgender Gleichung (6) bestimmt wird: ${Λ}_{m , acid} = 0.09921 \frac{mS l}{mmol cm} \frac{{c}_{{Na}^{+} , 0} + {c}_{{K}^{+} , 0} + 2 ∗ {c}_{{Mg}^{+} , 0} + 2 ∗ {c}_{{Ca}^{+} , 0}}{{c}_{{Na}^{+} , 0}}$ (6) wobei
Λ*_{m,acid}* dem unkorrigierten Parameter zur Umrechnung einer Natriumionenkonzentration in eine Leitfähigkeit der Dialysierflüssigkeit oder umgekehrt,
*c*_{*Na*⁺*,*0} einer Natriumionenkonzentration des verdünnten sauren Fluids,
*c*_{*K*⁺*,*0} einer Kaliumionenkonzentration des verdünnten sauren Fluids,
2 * *c*_{*Mg*⁺*,*0} einer Magnesiumionenkonzentration des verdünnten sauren Fluids und
2 * *c*_{*Ca*⁺,0} einer Calciumionenkonzentration des verdünnten sauren Fluids entspricht.

13. Verfahren nach Anspruch 10, **dadurch gekennzeichnet, dass** die Säurekonzentration des verdünnten sauren Fluids, dessen Natriumionenkonzentration eine Leitfähigkeit des verdünnten sauren Fluids von 11 mS/cm bewirkt, gemäß folgender Gleichung (7) bestimmt wird: ${c}_{acid} \left(11\frac{mS}{cm}\right) = \frac{{c}_{acid , 0} \left[\frac{mEq}{l}\right] ∗ {c}_{Na , {acid}^{+}} \left(11\frac{mS}{cm}\right)}{{c}_{{Na}^{+} , 0} - {c}_{bic , 0} - {c}_{acid , 0} \left[\frac{mEq}{l}\right]}$ (7) wobei
${c}_{acid} \left(11\frac{mS}{cm}\right)$ der Säurekonzentration des verdünnten sauren Fluids entspricht, dessen Natriumionenkonzentration eine Leitfähigkeit des verdünnten sauren Fluids von 11 mS/cm bewirkt,
*c*_{*bic,*0} einer Bicarbonationenkonzentration des verdünnten basischen Fluids entspricht,
*c*_{*acid,*0} einer Säurekonzentration des verdünnten sauren Fluids entspricht,
*c*_{*Na*⁺*,*0} einer Natriumionenkonzentration des verdünnten sauren Fluids entsprichtund
${c}_{Na , {acid}^{+}} \left(11\frac{mS}{cm}\right)$ einer Natriumionenkonzentration des verdünnten sauren Fluids entspricht, die eine Leitfähigkeit des verdünnten sauren Fluids von 11 mS/cm bewirkt.

14. Verfahren nach einem der vorhergehenden Ansprüche, **dadurch gekennzeichnet, dass** die Konzentrationen des basischen Fluids und des sauren Fluids auf einer Vorrichtung zur extrakorporalen Blutbehandlung, insbesondere zur Hämodialyse, gespeichert werden.

15. Verfahren nach einem der vorhergehenden Ansprüche, **dadurch gekennzeichnet, dass** das Verfahren wenigstens teilweise, insbesondere nur teilweise oder vollständig, von einer Vorrichtung zur extrakorporalen Blutbehandlung, insbesondere zur Hämodialyse, durchgeführt wird.

16. Verfahren nach einem der vorhergehenden Ansprüche, **dadurch gekennzeichnet, dass** abhängig von der gemäß Schritt i) bestimmten Bicarbonationenkonzentration eine weitere Zugabe des verdünnten basischen Fluids zu der Vorstufe der Dialysierflüssigkeit und/oder abhängig von der gemäß Schritt i) bestimmten Natriumionenkonzentration eine weitere Zugabe des verdünnten sauren Fluids zu der Dialysierflüssigkeit erfolgen/erfolgt.

17. Verwendung eines Verfahrens nach einem der vorhergehenden Ansprüche zur Herstellung oder Proportionierung der Dialysierflüssigkeit, insbesondere für die Hämodialyse.

18. Verwendung einer Vorrichtung zur extrakorporalen Blutbehandlung, insbesondere zur Hämodialyse, zur Durchführung eines Verfahrens nach einem der Ansprüche 1 bis 16.

## Claims

1. Method for determining a sodium and bicarbonate ion concentration of a dialysis fluid, the method comprising the following steps:
a) providing a dilute base fluid by mixing of a base fluid having a bicarbonate ion concentration with water according to a defined mixing ratio and providing a dilute acid fluid by mixing of an acid fluid having a sodium ion concentration and an acid concentration with water according to a defined mixing ratio,
b) preparing a pre-dialysis fluid by addition of the dilute base fluid to water,
c) determining a target value for a bicarbonate conductivity of the dialysis fluid and/or pre-dialysis fluid on the basis of a bicarbonate ion concentration for the dialysis fluid and/or pre-dialysis fluid and a parameter for converting a bicarbonate ion concentration into a bicarbonate conductivity or vice versa and determining a target value for a conductivity of the dialysis fluid on the basis of a sodium ion concentration for the dialysis fluid and a parameter for converting a sodium ion concentration into a conductivity of the dialysis fluid or vice versa,
d) measuring a conductivity of the pre-dialysis fluid,
e) comparing the conductivity of the pre-dialysis fluid measured according to step d) with the target value for the bicarbonate conductivity of the dialysis fluid and/or pre-dialysis fluid determined according to step c) and, if necessary, carrying out further addition of the dilute base fluid to the pre-dialysis fluid until the target value for the bicarbonate conductivity of the dialysis fluid and/or pre-dialysis fluid has been reached or substantially reached,
f) preparing the dialysis fluid by addition of the dilute acid fluid to the pre-dialysis fluid,
g) measuring a conductivity of the dialysis fluid,
h) comparing the conductivity of the dialysis fluid measured according to step g) with the target value for the conductivity of the dialysis fluid determined according to step c) and, if necessary, carrying out further addition of the dilute acid fluid to the dialysis fluid until the target value for the conductivity of the dialysis fluid has been reached or substantially reached, and
i) determining the bicarbonate ion concentration of the dialysis fluid on the basis of the conductivity of the pre-dialysis fluid measured according to step d) and the parameter for converting a bicarbonate ion concentration into a bicarbonate conductivity or vice versa and determining the sodium ion concentration of the dialysis fluid on the basis of the conductivity of the dialysis fluid measured according to step g) and the parameter for converting a sodium ion concentration into a conductivity of the dialysis fluid or vice versa,
wherein the parameter for converting a bicarbonate ion concentration into a bicarbonate conductivity or vice versa and the parameter for converting a sodium ion concentration into a conductivity of the dialysis fluid or vice versa are both ascertained automatically.

2. Method according to Claim 1, **characterized in that** the base fluid also has a sodium ion concentration, in particular is provided in the form of an aqueous basic solution comprising sodium hydrogencarbonate.

3. Method according to Claim 1 or 2, **characterized in that** the acid fluid also has a potassium ion concentration, a magnesium ion concentration and a calcium ion concentration, in particular is provided in the form of an aqueous acidic solution comprising sodium chloride, potassium chloride, magnesium chloride, calcium chloride and an acid, in particular acetic acid.

4. Method according to any of the preceding claims, **characterized in that** the target value for the bicarbonate conductivity of the dialysis fluid and/or pre-dialysis fluid is determined with the aid of a linear model, in particular according to the following equation (1): $BICLF = {c}_{bic} \left(mmol/l\right) \times {Λ}_{m , bic}$ (1) v
BICLF is the target value for the bicarbonate conductivity of the dialysis fluid and/or pre-dialysis fluid,
c_{bic} is the bicarbonate ion concentration for the dialysis fluid and/or pre-dialysis fluid and
Λ_{*m*,*bic*} is the parameter for converting a bicarbonate ion concentration into a bicarbonate conductivity or vice versa.

5. Method according to any of the preceding claims, **characterized in that** the target value for the conductivity of the dialysis fluid is determined with the aid of a linear model, in particular according to the following equation (2): 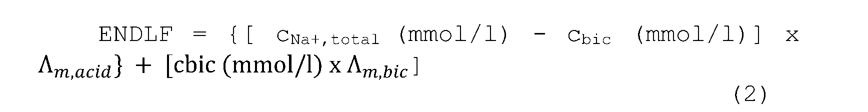 ENDLF = {[cNa+,total (mmol/l) - cbic (mmol/l)] x Λm,acid} + [cbic (mmol/l) x Λm,bic] (2) where
ENDLF is the target value for the conductivity of the dialysis fluid,
c_{Na+,totat} is the sodium ion concentration for the dialysis fluid,
c_{bic} is the bicarbonate ion concentration for the dialysis fluid and/or pre-dialysis fluid,
Λ*_{m,acid}* is the parameter for converting a sodium ion concentration into a conductivity of the dialysis fluid or vice versa
and
Λ*_{m,bic}* is the parameter for converting a bicarbonate ion concentration into a bicarbonate conductivity or vice versa.

6. Method according to any of the preceding claims, **characterized in that** the parameter for converting a bicarbonate ion concentration into a bicarbonate conductivity or vice versa and the parameter for converting a sodium ion concentration into a conductivity of the dialysis fluid or vice versa are ascertained automatically on the basis of concentrations of the dilute base fluid and/or the dilute acid fluid, preferably the dilute acid fluid.

7. Method according to any of the preceding claims, **characterized in that** the parameter for converting a bicarbonate ion concentration into a bicarbonate conductivity or vice versa is ascertained automatically on the basis of a bicarbonate ion concentration of the dilute base fluid and an acid concentration of the dilute acid fluid.

8. Method according to any of the preceding claims, **characterized in that** the parameter for converting a sodium ion concentration into a conductivity of the dialysis fluid or vice versa is ascertained automatically on the basis of a bicarbonate ion concentration of the dilute base fluid, a sodium ion concentration of the dilute base fluid, a sodium ion concentration of the dilute acid fluid, a potassium ion concentration of the dilute acid fluid, a magnesium ion concentration of the dilute acid fluid, a calcium ion concentration of the dilute acid fluid and an acid concentration of the dilute acid fluid.

9. Method according to any of the preceding claims, **characterized in that** the parameter for converting a bicarbonate ion concentration into a bicarbonate conductivity or vice versa is determined according to the following equation (3): ${Λ}_{m , bic} = 0.0852 \frac{mS l}{mmol cm} \frac{{c}_{bic , 0} + {c}_{acid , 0} \left[\frac{mEq}{l}\right]}{{c}_{bic , 0}}$ (3) where
Λ*_{m,bic}* is the parameter for converting a bicarbonate ion concentration into a bicarbonate conductivity or vice versa,
*c*_{*bic,*0} is a bicarbonate ion concentration of the dilute base fluid and
*c*_{*acid,*0} is an acid concentration of the dilute acid fluid.

10. Method according to any of the preceding claims, **characterized in that** the parameter for converting a sodium ion concentration into a conductivity of the dialysis fluid or vice versa is determined according to the following equation (4): ${Λ}_{m , acid , kor} = \frac{11 \frac{mS}{cm}}{{c}_{Na , {acid}^{+}} \left(11\frac{mS}{cm}\right) + {c}_{acid} \left(11\frac{mS}{cm}\right)}$ (4) where
Λ*_{m,acid,kor}* is the parameter for converting a sodium ion concentration into a conductivity of the dialysis fluid or vice versa,
${c}_{Na , {acid}^{+}} \left(11\frac{mS}{cm}\right)$ is a sodium ion concentration of the dilute acid fluid that brings about a conductivity of the dilute acid fluid of 11 mS/cm, and
${c}_{acid} \left(11\frac{mS}{cm}\right)$ is an acid concentration of the dilute acid fluid having a sodium ion concentration which brings about a conductivity of the dilute acid fluid of 11 mS/cm.

11. Method according to Claim 10, **characterized in that** the sodium ion concentration of the dilute acid fluid that brings about a conductivity of the dilute acid fluid of 11 mS/cm is determined according to the following equation (5): ${c}_{Na , {acid}^{+}} \left(11\frac{mS}{cm}\right) = \frac{11 \frac{mS}{cm}}{{Λ}_{m , acid}}$ (5) where
${c}_{Na , {acid}^{+}} \left(11\frac{mS}{cm}\right)$ is the sodium ion concentration of the dilute acid fluid that brings about a conductivity of the dilute acid fluid of 11 mS/cm,and
**Λ*_{m,acid}*** is an uncorrected parameter for converting a sodium ion concentration into a conductivity of the dialysis fluid or vice versa.

12. Method according to Claim 11, **characterized in that** the uncorrected parameter **Λ*_{m,acid}*** for converting a sodium ion concentration into a conductivity of the dialysis fluid or vice versa is determined according to the following equation (6): ${Λ}_{m , acid} = 0.09921 \frac{mS l}{mmol cm} \frac{{c}_{{Na}^{+} , 0} + {c}_{{K}^{+} , 0} + 2 ∗ {c}_{{Mg}^{+} , 0} + 2 ∗ {c}_{{Ca}^{+} , 0}}{{c}_{{Na}^{+} , 0}}$ (6) where
**Λ*_{m,acid}*** is the uncorrected parameter for converting a sodium ion concentration into a conductivity of the dialysis fluid or vice versa,
*c*_{*Na*⁺*,*0} is a sodium ion concentration of the dilute acid fluid,
*c*_{*K*⁺*,*0} is a potassium ion concentration of the dilute acid fluid,
2 * *c*_{*Mg*⁺*,*0} is a magnesium ion concentration of the dilute acid fluid
and
2 * *c*_{*Ca*⁺*,*0} is a calcium ion concentration of the dilute acid fluid.

13. Method according to Claim 10, **characterized in that** the acid concentration of the dilute acid fluid having a sodium ion concentration which brings about a conductivity of the dilute acid fluid of 11 mS/cm is determined according to the following equation (7): ${c}_{acid} \left(11\frac{mS}{cm}\right) = \frac{{c}_{acid , 0} \left[\frac{mEq}{l}\right] ∗ {c}_{Na , {acid}^{+}} \left(11\frac{mS}{cm}\right)}{{c}_{{Na}^{+} , 0} - {c}_{bic , 0} - {c}_{acid , 0} \left[\frac{mEq}{l}\right]}$ (7) where
${c}_{acid} \left(11\frac{mS}{cm}\right)$ is the acid concentration of the dilute acid fluid having a sodium ion concentration which brings about a conductivity of the dilute acid fluid of 11 mS/cm,
*c*_{*bic*,0} is a bicarbonate ion concentration of the dilute base fluid,
*c*_{*acid*,0} is an acid concentration of the dilute acid fluid,
*c*_{*Na*⁺*,*0} is a sodium ion concentration of the dilute acid fluid
and
${c}_{Na , {acid}^{+}} \left(11\frac{mS}{cm}\right)$ is a sodium ion concentration of the dilute acid fluid that brings about a conductivity of the dilute acid fluid of 11 mS/cm.

14. Method according to any of the preceding claims, **characterized in that** the concentrations of the base fluid and the acid fluid are stored on an extracorporeal blood treatment device, in particular a haemodialysis device.

15. Method according to any of the preceding claims, **characterized in that** at least part of the method, in particular only part of the method or the entire method, is performed by an extracorporeal blood treatment device, in particular a haemodialysis device.

16. Method according to any of the preceding claims, **characterized in that**, depending on the bicarbonate ion concentration determined according to step i), a further addition of the dilute base fluid to the pre-dialysis fluid is carried out and/or, depending on the sodium ion concentration determined according to step i), a further addition of the dilute acid fluid to the dialysis fluid is carried out.

17. Use of a method according to any of the preceding claims for preparation or proportioning of the dialysis fluid, in particular for haemodialysis.

18. Use of an extracorporeal blood treatment device, in particular a haemodialysis device, for performance of a method according to any of Claims 1 to 16.

## Revendications

1. Procédé de détermination d'une concentration en ions sodium et en ions bicarbonate d'un liquide de dialyse, le procédé comprenant les étapes suivantes :
a) la fourniture d'un fluide basique dilué par mélange d'un fluide basique présentant une concentration en ions bicarbonate avec de l'eau selon un rapport de mélange défini, et la fourniture d'un fluide acide dilué par mélange d'un fluide acide présentant une concentration en ions sodium ainsi qu'une concentration en acide avec de l'eau selon un rapport de mélange défini,
b) la préparation d'un précurseur du liquide de dialyse par ajout du fluide basique dilué à de l'eau,
c) la détermination d'une valeur de consigne d'une conductivité de bicarbonate du liquide de dialyse et/ou du précurseur du liquide de dialyse sur la base d'une concentration en ions bicarbonate pour le liquide de dialyse et/ou le précurseur du liquide de dialyse et d'un paramètre permettant de convertir une concentration en ions bicarbonate en une conductivité de bicarbonate ou inversement, et la détermination d'une valeur de consigne d'une conductivité du liquide de dialyse sur la base d'une concentration en ions sodium pour le liquide de dialyse et d'un paramètre permettant de convertir une concentration en ions sodium en une conductivité du liquide de dialyse ou inversement,
d) la mesure d'une conductivité du précurseur du liquide de dialyse,
e) la comparaison de la conductivité du précurseur du liquide de dialyse mesurée à l'étape d) avec la valeur de consigne de la conductivité de bicarbonate du liquide de dialyse et/ou du précurseur du liquide de dialyse déterminée à l'étape c), et, le cas échéant, l'ajout supplémentaire du fluide basique dilué au précurseur du liquide de dialyse jusqu'à ce que la valeur de consigne de la conductivité de bicarbonate du liquide de dialyse et/ou du précurseur du liquide de dialyse soit atteinte ou sensiblement atteinte,
f) la préparation du liquide de dialyse par ajout du fluide acide dilué au précurseur du liquide de dialyse,
g) la mesure d'une conductivité du liquide de dialyse,
h) la comparaison de la conductivité du liquide de dialyse mesurée à l'étape g) avec la valeur de consigne de la conductivité du liquide de dialyse déterminée à l'étape c), et, le cas échéant, l'ajout supplémentaire du fluide acide dilué au liquide de dialyse jusqu'à ce que la valeur de consigne de la conductivité du liquide de dialyse soit atteinte ou sensiblement atteinte, et
i) la détermination de la concentration en ions bicarbonate du liquide de dialyse sur la base de la conductivité du précurseur du liquide de dialyse mesurée à l'étape d) et du paramètre permettant de convertir une concentration en ions bicarbonate en une conductivité de bicarbonate ou inversement, ainsi que la détermination de la concentration en ions sodium du liquide de dialyse sur la base de la conductivité du liquide de dialyse mesurée à l'étape g) et du paramètre permettant de convertir une concentration en ions sodium en une conductivité du liquide de dialyse ou inversement,
dans lequel le paramètre permettant de convertir une concentration en ions bicarbonate en une conductivité de bicarbonate ou inversement et le paramètre permettant de convertir une concentration en ions sodium en une conductivité du liquide de dialyse ou inversement sont chacun déterminés automatiquement.

2. Procédé selon la revendication 1, **caractérisé en ce que** le fluide basique présente en outre une concentration en ions sodium, et est notamment fourni sous la forme d'une solution basique aqueuse comprenant de l'hydrogénocarbonate de sodium.

3. Procédé selon la revendication 1 ou 2, **caractérisé en ce que** le fluide acide présente en outre une concentration en ions potassium, une concentration en ions magnésium et une concentration en ions calcium, et est notamment fourni sous la forme d'une solution acide aqueuse comprenant du chlorure de sodium, du chlorure de potassium, du chlorure de magnésium, du chlorure de calcium et un acide, en particulier de l'acide acétique.

4. Procédé selon l'une quelconque des revendications précédentes, **caractérisé en ce que** la valeur de consigne de la conductivité de bicarbonate du liquide de dialyse et/ou du précurseur du liquide de dialyse est déterminée à l'aide d'un modèle linéaire, en particulier selon l'équation (1) suivante : $BICLF = {c}_{bic} \left(mmol/l\right) \times {Λ}_{m , bic}$ (1) dans laquelle
BICLF représente la valeur de consigne de la conductivité de bicarbonate du liquide de dialyse et/ou du précurseur du liquide de dialyse,
c_{bic} représente la concentration en ions bicarbonate pour le liquide de dialyse et/ou le précurseur du liquide de dialyse,
et
Λ_{m,bic} représente le paramètre permettant de convertir une concentration en ions bicarbonate en une conductivité de bicarbonate ou inversement.

5. Procédé selon l'une quelconque des revendications précédentes, **caractérisé en ce que** la valeur de consigne de la conductivité du liquide de dialyse est déterminée à l'aide d'un modèle linéaire, en particulier selon l'équation (2) suivante : 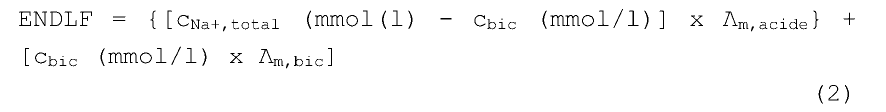 ENDLF = {[cNa+,total (mmol(l) - cbic (mmol/l)] x Λm,acide} + [cbic (mmol/l) x Λm,bic] (2) dans laquelle
ENDLF représente la valeur de consigne de la conductivité du liquide de dialyse,
c_{Na+,total} représente la concentration en ions sodium pour le liquide de dialyse,
c_{bic} représente la concentration en ions bicarbonate pour le liquide de dialyse et/ou le précurseur du liquide de dialyse,
Λ_{m,acide} représente le paramètre permettant de convertir une concentration en ions sodium en une conductivité du liquide de dialyse ou inversement,
et
Λ_{m,bic} représente le paramètre permettant de convertir une concentration en ions bicarbonate en une conductivité de bicarbonate ou inversement.

6. Procédé selon l'une quelconque des revendications précédentes, **caractérisé en ce que** le paramètre permettant de convertir une concentration en ions bicarbonate en une conductivité de bicarbonate ou inversement ainsi que le paramètre permettant de convertir une concentration en ions sodium en une conductivité du liquide de dialyse ou inversement sont déterminés automatiquement sur la base de concentrations du fluide basique dilué et/ou du fluide acide dilué, de préférence du fluide acide dilué.

7. Procédé selon l'une quelconque des revendications précédentes, **caractérisé en ce que** le paramètre permettant de convertir une concentration en ions bicarbonate en une conductivité de bicarbonate ou inversement est déterminé automatiquement sur la base d'une concentration en ions bicarbonate du fluide basique dilué et d'une concentration en acide du fluide acide dilué.

8. Procédé selon l'une quelconque des revendications précédentes, **caractérisé en ce que** le paramètre permettant de convertir une concentration en ions sodium en une conductivité du liquide de dialyse ou inversement est déterminé automatiquement sur la base d'une concentration en ions bicarbonate du fluide basique dilué, d'une concentration en ions sodium du fluide basique dilué, d'une concentration en ions sodium du fluide acide dilué, d'une concentration en ions potassium du fluide acide dilué, d'une concentration en ions magnésium du fluide acide dilué, d'une concentration en ions calcium du fluide acide dilué et d'une concentration en acide du fluide acide dilué.

9. Procédé selon l'une quelconque des revendications précédentes, **caractérisé en ce que** le paramètre permettant de convertir une concentration en ions bicarbonate en une conductivité de bicarbonate ou inversement est déterminé selon l'équation (3) suivante : ${Λ}_{m , bic} = 0 , 0852 \frac{mS l}{mmol cm} \frac{{c}_{bic , 0} + {c}_{acide , 0} \left[\frac{mEq}{l}\right]}{{c}_{bic , 0}}$ (3) dans laquelle
Λ_{m,bic} représente le paramètre permettant de convertir une concentration en ions bicarbonate en une conductivité de bicarbonate ou inversement,
*c*_{*bic*,0} représente une concentration en ions bicarbonate du fluide basique dilué,
et
*c*_{*acide*,0} représente une concentration en acide du fluide acide dilué.

10. Procédé selon l'une quelconque des revendications précédentes, **caractérisé en ce que** le paramètre permettant de convertir une concentration en ions sodium en une conductivité du liquide de dialyse ou inversement est déterminé selon l'équation (4) suivante : ${Λ}_{m , acid , cor} = \frac{11 \frac{mS}{cm}}{{c}_{Na , {acide}^{+}} \left(11\frac{mS}{cm}\right) + {c}_{acide} \left(11\frac{mS}{cm}\right)}$ (4) dans laquelle
Λ_{m,*acid,cor*} Le paramètre de conversion d'une concentration en ions sodium en une conductivité du liquide de dialyse, ou vice versa,
${c}_{Na , {acide}^{+}} \left(11\frac{mS}{cm}\right)$ représente une concentration en ions sodium du fluide acide dilué qui entraîne une conductivité du fluide acide dilué de 11 mS/cm,
et
${c}_{acide} \left(11\frac{mS}{cm}\right)$ représente une concentration en acide du fluide acide dilué dont la concentration en ions sodium entraîne une conductivité du fluide acide dilué de 11 mS/cm.

11. Procédé selon la revendication 10, **caractérisé en ce que** la concentration en ions sodium du fluide acide dilué qui entraîne une conductivité du fluide acide dilué de 11 mS/cm est déterminée selon l'équation (5) suivante : ${c}_{Na , {acid}^{+}} \left(11\frac{mS}{cm}\right) = \frac{11 \frac{mS}{cm}}{{Λ}_{m , acide}}$ (5) dans laquelle
${c}_{Na , {acid}^{+}} \left(11\frac{mS}{cm}\right)$ représente la concentration en ions sodium du fluide acide dilué qui entraîne une conductivité du fluide acide dilué de 11 mS/cm,
et
Λ*_{m,acide}* représente un paramètre non corrigé permettant de convertir une concentration en ions sodium en une conductivité du liquide de dialyse ou inversement.

12. Procédé selon la revendication 11, **caractérisé en ce que** le paramètre non corrigé Λ_{m,acid} permettant de convertir une concentration en ions sodium en une conductivité du liquide de dialyse ou inversement est déterminé selon l'équation (6) suivante : ${Λ}_{m , acide} = 0 , 09921 \frac{mS l}{mmol cm} \frac{{c}_{{Na}^{+} , 0} + {c}_{{K}^{+} , 0} + 2 ∗ {c}_{{Mg}^{+} , 0} + 2 ∗ {c}_{{Ca}^{+} , 0}}{{c}_{{Na}^{+} , 0}}$ (6) dans laquelle
Λ_{m,acide} représente le paramètre non corrigé permettant de convertir une concentration en ions sodium en une conductivité du liquide de dialyse ou inversement,
*c*_{*Na*⁺*,*0} représente une concentration en ions sodium du fluide acide dilué,
*c*_{*K*⁺*,*0} représente une concentration en ions potassium du fluide acide dilué,
2 ** c*_{*Mg*⁺*,*0} représente une concentration en ions magnésium du fluide acide dilué,
et
2 * *c*_{*Ca*⁺*,*0} représente une concentration en ions calcium du fluide acide dilué.

13. Procédé selon la revendication 10, **caractérisé en ce que** la concentration en acide du fluide acide dilué dont la concentration en ions sodium entraîne une conductivité du fluide acide dilué de 11 mS/cm est déterminée selon l'équation (7) suivante : ${c}_{acide} \left(11\frac{mS}{cm}\right) = \frac{{c}_{acide , 0} \left[\frac{mEq}{l}\right] ∗ {c}_{Na , {acide}^{+}} \left(11\frac{mS}{cm}\right)}{{c}_{{Na}^{+} , 0} - {c}_{bic , 0} - {c}_{acide , 0} \left[\frac{mEq}{l}\right]}$ (7) dans laquelle
${c}_{acide} \left(11\frac{mS}{cm}\right)$ représente la concentration en acide du fluide acide dilué dont la concentration en ions sodium entraîne une conductivité du fluide acide dilué de 11 mS/cm,
*c*_{*bic*,0} représente une concentration en ions bicarbonate du fluide basique dilué,
*c*_{*acide*,0} représente une concentration en acide du fluide acide dilué,
*c*_{*Na*⁺*,*0} représente une concentration en ions sodium du fluide acide dilué,
et
${c}_{Na , {acide}^{+}} \left(11\frac{mS}{cm}\right)$ représente une concentration en ions sodium du fluide acide dilué qui entraîne une conductivité du fluide acide dilué de 11 mS/cm.

14. Procédé selon l'une quelconque des revendications précédentes, **caractérisé en ce que** les concentrations du fluide basique et du fluide acide sont stockées dans un dispositif de traitement extracorporel du sang, en particulier d'hémodialyse.

15. Procédé selon l'une quelconque des revendications précédentes, **caractérisé en ce que** le procédé est mis en œuvre au moins partiellement, en particulier seulement partiellement ou intégralement, par un dispositif de traitement extracorporel du sang, en particulier d'hémodialyse.

16. Procédé selon l'une quelconque des revendications précédentes, **caractérisé en ce que**, en fonction de la concentration en ions bicarbonate déterminée à l'étape i), un ajout supplémentaire du fluide basique dilué au précurseur du liquide de dialyse est effectué et/ou, en fonction de la concentration en ions sodium déterminée à l'étape i), un ajout supplémentaire du fluide acide dilué au liquide de dialyse est effectué.

17. Utilisation d'un procédé selon l'une quelconque des revendications précédentes pour la préparation ou le dosage proportionnel du liquide de dialyse, en particulier pour l'hémodialyse.

18. Utilisation d'un dispositif de traitement extracorporel du sang, en particulier d'hémodialyse, pour la mise en œuvre d'un procédé selon l'une quelconque des revendications 1 à 16.
